# EUROPEAN PATENT SPECIFICATION

(11) **EP 4 497 833 B1**
(45) Date of publication and mention of the grant of the patent: **26.08.2026**
(21) Application number: 24207925.9
(22) Date of filing: 19.06.2020
(51) Int. Cl.: C12Q 1/6816

(54) **TYPE III CRISPR/CAS-BASED DIAGNOSTICS**
TYP-III-CRISPR/CAS-BASIERTE DIAGNOSTIK
DIAGNOSTIC SUR LA BASE DE CRISPR/CAS DE TYPE III

(30) Priority: 19.06.2019 EP 19181371; 07.04.2020 EP 20168487
(43) Date of publication of application: 29.01.2025
(62) Document number(s) of the earlier application(s) in accordance with Art. 76 EPC: 20744159.3 / 3 987 058
(73) Proprietor: SCOPE BIOSCIENCES B.V., 6708 WH Wageningen (NL)
(72) Inventor: STEENS, Aiko Jurre, 6721 BK Bennekom (NL); PRINSEN, Stijn Hendrikus Petrus, 6708 PG Wageningen (NL); VAN DER OOST, John, 6871 HZ Renkum (NL); STAALS, Raymond Hubert Josèphe, 6711NZ Ede (NL)
(74) Representative: HGF

(56) References cited:
- JONATHAN S. GOOTENBERG ET AL: "Multiplexed and portable nucleic acid detection platform with Cas13, Cas12a, and Csm6", SCIENCE, vol. 360, no. 6387, 15 February 2018 (2018-02-15), US, pages 439 - 444, XP055538780, ISSN: 0036-8075, DOI: 10.1126/science.aaq0179
- CHRISTOPHE ROUILLON ET AL: "Control of cyclic oligoadenylate synthesis in a type III CRISPR system", ELIFE, vol. 7, 2 July 2018 (2018-07-02), XP055729022, DOI: 10.7554/eLife.36734
- OLE NIEWOEHNER ET AL: "Type III CRISPR-Cas systems produce cyclic oligoadenylate second messengers", NATURE, vol. 548, no. 7669, 19 July 2017 (2017-07-19), London, pages 543 - 548, XP055495137, ISSN: 0028-0836, DOI: 10.1038/nature23467
- HAN WENYUAN ET AL: "A Type III-B Cmr effector complex catalyzes the synthesis of cyclic oligoadenylate second messengers by cooperative substrate binding", vol. 46, no. 19, 1 January 2018 (2018-01-01), GB, XP093038213, ISSN: 0305-1048, Retrieved from the Internet <URL:https://www.ncbi.nlm.nih.gov/pmc/articles/PMC6212834/pdf/gky844.pdf> DOI: 10.1093/nar/gky844
- PAN SAIFU ET AL: "A seed motif for target RNA capture enables efficient immune defence by a type III-B CRISPR-Cas system", RNA BIOLOGY, vol. 16, no. 9, 26 May 2019 (2019-05-26), pages 1166 - 1178, XP093038262, ISSN: 1547-6286, DOI: 10.1080/15476286.2019.1618693

## Description

FIELD: The invention described herein is directed to a CRISPR/Cas-related nucleic acid detection system and its broad use in diagnostic applications.

### 1. INTRODUCTION

Over the past decade, the world of molecular biology has been subjected to several ground-breaking discoveries that have had consequences reaching far beyond its own field. Undoubtedly, CRISPR/Cas (Clustered Regularly Interspaced Short Palindromic Repeats, CRISPR/associated genes) can be considered as one of these discoveries. The CRISPR/Cas system was identified as a prokaryotic adaptive immune system, providing sequence-specific defense against foreign genetic elements, like bacteriophages and plasmids. However, the CRISPR/Cas system allows genetic perturbations in most, if not all, organisms, which will have enormous consequences in all fields of technology.

CRISPR defense can be described as a process consisting of three stages: adaptation, expression, and interference (Rath et al., 2015. Biochimie 117: 119-128; Makarova et al., 2011. Nat Rev Microbiol 9: 467-477). During the adaptation stage, genetic fragments are acquired from foreign invading entities and stored in the CRISPR memory (Jackson et al., 2017. Science 356: eaal5056). This memory comprises the foreign DNA sequences, called spacers, which are separated by repetitive DNA sequences (repeats). Expression of the CRISPR locus (stage II) leads to transcription of a long RNA molecule which is subsequently processed into multiple CRISPR RNAs (crRNAs) (Brouns et al., 2008. Science 321: 960-964). Additionally, the cell expresses Cas proteins, the effectors of the CRISPR/Cas system, which form ribonucleoprotein (RNP) complexes by incorporating the crRNAs. Once a foreign genetic element is detected due to sequence complementarity to a crRNA, the associated Cas protein will use its nuclease activity to degrade the invading entity, often described as the interference stage of the CRISPR defense process.

Due to the vast amount of research dedicated to CRISPR/Cas over the past decade, a large number of systems has been discovered within the kingdoms of bacteria and archaea (Fenner et al., 2007. J Biomol Screen 20: 1027-1039; van der Oost et al., 2009. Trends Biochem Sciences 34: 401-407). A categorization of CRISPR/Cas systems has been made. Class I systems utilize multisubunit Cas complexes, whereas the class II systems use only a single Cas protein to mediate its activity. Different types are generally characterized based on the presence of signature genes (Wright et al., 2016. Cell 164: 29-44).

In contrast to the differences that make it possible to distinguish the many CRISPR/Cas systems, the vast majority share functional similarities. Nearly all CRISPR/Cas types function as DNA targeting RNPs, which is likely related to the large number of DNA-based invading entities. In contrast, invading elements of an RNA-based nature are not often encountered in the prokaryotic world. It is therefore somewhat surprising that Type III CRISPR/Cas systems have evolved to target RNA sequences (Wright et al., 2016. Cell 164: 29-44; Hale et al., 2009. Cell 139: 945-956). Research has attributed this aberrant activity to the degradation of transcripts originating from invading bacteriophages, thereby preventing them from lysing the cell (Jiang et al., 2016. Cell 164: 710-721; Goldberg et al., 2014. Nature 514: 633-637). The Type III system belongs to class I, meaning that RNPs consist of multiple subunits.

With three different types of nuclease activity, the Type III system can be considered unique. Rouillon *et al.* (Rouillon et al., 2018. eLIFE 7: e36734) describes a Type IIID Cas-mediated effector system of *S. solfataricus.* Niewoehner *et al.* (Niewoehner et al., 2017. Nature 548: 543-548) describes a Type IIIA Cas effector system of *Staphylococcus epidermidis.* Han *et al.* (Han et al., 2018. Nucleic Acids Research 46: 10319-10330) describes a Type IIIB Cmr effector complex. Pan Saifu *et al. (*Pan et al., 2019. RNA Biol. 16(9):1166-117***)*** describes a seed motif for target RNA capture which enables efficient immune defence by a type III-B CRISPR-Cas system. WO 2018/220583 A1 discloses the production of cyclic adenylates and their use as allosteric regulators. Jonathon S. Gootenburg *et al.* (Gootenburg et al., 2017. Science. 360(6387): 439-444***)*** describes a multiplexed and portable nucleic acid detection platform with Cas13, Cas12a, and Csm6. Furthermore, the production of the messenger molecule cyclic oligoadenylate (cOA) has not been ascribed to any other CRISPR/Cas system. Exploiting these features could lead to new ways to visualize target recognition, which makes the Type III system suitable for application in novel diagnostic tools.

### 2. BRIEF DESCRIPTION OF THE INVENTION

The invention is defined by the claims. Further aspects and preferred embodiments are defined in the dependent claims. Any aspects, embodiments and examples of the present disclosure which do not fall under the scope of the appended claims do not form part of the invention and are merely provided for illustrative purposes.

Accordingly, the invention provides a method for determining presence or absence of a target nucleic acid molecule in a sample, wherein the method is for detection of bacterial, fungal, archaeal, protest, protozoal, eukaryotic, viral and viroidal pathogens, for detection of beneficial organisms, or for detection and diagnosis of genetic alterations or traits that are expressed as RNA molecules, in human, animals and plants, comprising:
amplification of the target nucleic acid molecule present in the sample, and providing the sample with a clustered regularly interspaced short palindromic repeats (CRISPR) based ribonucleic acid detection system derived comprising:
   a) an effector complex comprising a Type III CRISPR-associated effector protein (Cas) and at least one CRISPR RNA (crRNA) that binds to a target nucleic acid molecule;
   b) means for directly or indirectly determining a level of cyclic oligoadenylate (COA);
incubating the sample under conditions that allow binding of the crRNA to its target nucleic acid molecule; and
directly or indirectly determining a level of cyclic oligoadenylate (cOA), whereby an increase in the determined cOA level, as compared to a control, is indicative of the presence of said target molecule in said sample.

Described herein is a clustered regularly interspaced short palindromic repeats (CRISPR) based ribonucleic acid detection system comprising a) an effector complex comprising a Type III CRISPR-associated effector protein (Cas) and at least one CRISPR RNA (crRNA) that binds to a target nucleic acid molecule, and b) means for directly or indirectly determining a level of cyclic oligoadenylate (cOA). Said detection system preferably is a Type III Cas is a Type IIIB Cas, preferably a Type IIIB Cmr. Said Type III Cas preferably is from a thermophylic organism such as *Thermus thermophilus.*

A Type III Cas preferably lacks cleavage activity, for example by a D26N mutation of a Cmr4 subunit, or an equivalent mutation in another subunit. Further preferred cleavage-dead mutations include Cmr4 D26A, a E227A and E228 double mutant of Cmr4, and Cmr4 D86A (Ramia et al., 2014. Cell Reports 9: 1610-1617; Zhu and Ye, 2015. Nucleic Acids Res 43: 1257-1267). In addition, a Csm3 D32A is a good candidate for a cleavage-dead Type IIIA Csm mutant (Samai et al., 2015. Cell 161: 1164-1174).

In a preferred detection system disclosed herein, said means for directly or indirectly determining a level of cOA comprise means for determining a level of pyrophosphate (PPi). A preferred detection system according to the invention may further comprise an inorganic pyrophosphatase. Said inorganic pyrophosphatase preferably is from a thermophylic organism such as *Thermus thermophilus,* making it compatible with the preferred Type III CRISPR/Cas system and allowing isothermal detection.

A preferred detection system may further comprise a cOA-dependent, non-specific effector endoribonuclease such as Csx1

A preferred detection system according to the invention further comprises a cOA-dependent, non-specific effector endoribonuclease such as Csx1, and a detectable substrate for said endoribonuclease.

Further provided herein is a method for determining presence or absence of a target nucleic acid molecule in a sample, comprising providing the sample with a ribonucleic acid detection system according to the invention, incubating the sample under conditions that allow binding of the crRNA to its target nucleic acid molecule, and directly or indirectly determining a level of cyclic oligoadenylate (cOA), whereby an increase in the determined cOA level, as compared to a control, is indicative of the presence of said target molecule in said sample.

A level of cOA may be determined by determining a level of pyrophosphate, or a level of inorganic phosphate in case an inorganic pyrophosphatase is present in the detection system. A level of pyrophosphate or inorganic phosphate preferably is determined by a colorimetric-, fluorometric-, fluorescent- or bioluminescent-based assay.

A level of cOA may indirectly be determined by determining a level of a cOA-dependent, non-specific effector endoribonuclease such as Csx1 by detecting a detectable substrate for said effector endoribonuclease.

A preferred method disclosed herein comprises incubating the sample with the ribonucleic acid detection system at a temperature between 37 °C and 85 °C, preferably at about 65 °C.

Further provided herein is a device comprising the ribonucleic acid detection system according to the invention. Said device preferably comprises multiple arrayed ribonucleic acid detection systems according to the invention. Said multiple arrayed ribonucleic acid detection systems preferably have different target nucleic acid molecules.

Further provided herein is a kit of parts comprising a) an effector complex comprising a Type III CRISPR-associated effector protein (Cas) and at least one CRISPR RNA (crRNA) that binds to a target nucleic acid molecule, and b) means for directly or indirectly determining a level of cyclic oligoadenylate (cOA).

### 3. FIGURE LEGENDS

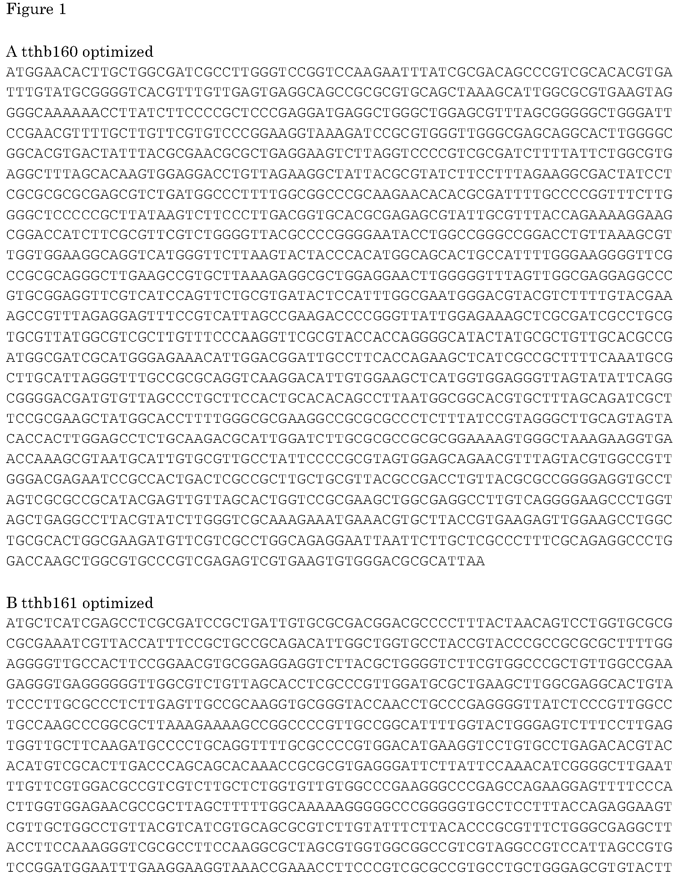

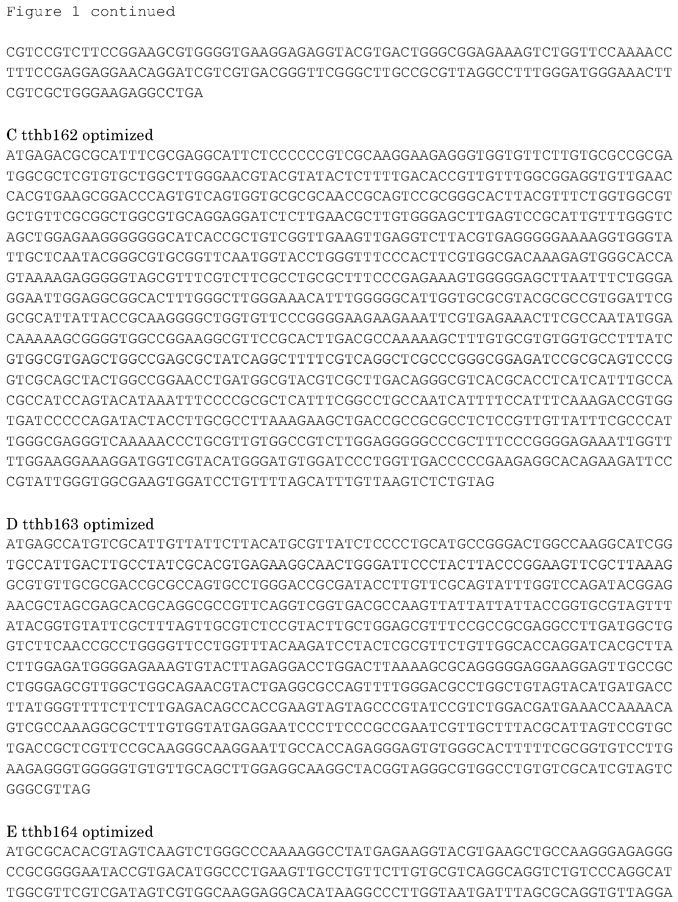

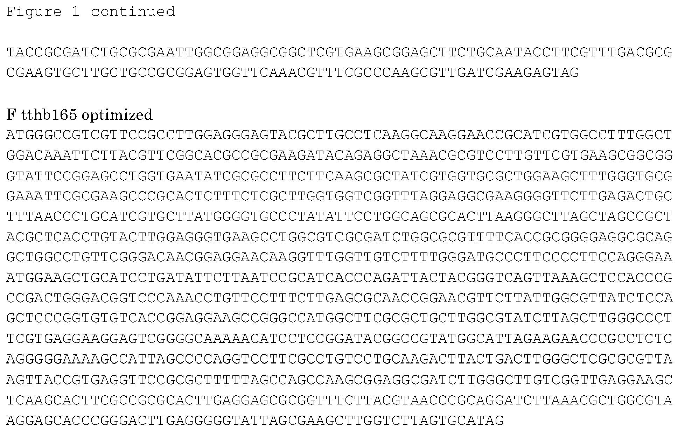

Figure 1. Codon optimized Cmr Cas proteins using IDT Codon Optimization Tool (available at eu.idtdna.com/codonopt).

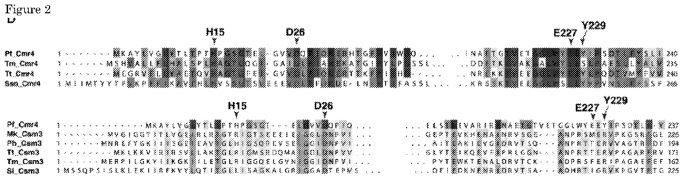

Figure 2. Alignment of Cmr and Csm sequences.
Figure 3. In vitro RNase activity assays with the endogenous and reconstituted TtCmr complexes. (A) Denaturing polyacrylamide gel electrophoresis (PAGE) analysis of the activity assay using a 5'- phosphorus-32 labeled target RNA complementary to the crRNA incubated with the endogenous TtCmr complex. A single stranded RNA marker ("M") was used as size standards as indicated on the left. (B) Activity assays similar to panel A, but using the reconstituted complexes.
Figure 4. (A) RNAse activity of TtCmr is not affected by targets with mismatches in the first guide nucleotides of crRNA. (B) cOA production is significantly affected by mismatches at nucleotide positions 1, 2 and 5.
Figure 5. cOA production assay with the A) TtCmr40 and B) TtCmr46, incubated with target RNAs (Table 4) with mismatches at the indicated nucleotide positions.
Figure 6. A flexible seed region at the 3' end of the crRNA. Target RNA degradation and cOA production assay with the (A) endogenous TtCmr ("TtCmr) or the reconstituted TtCmr complex with the (B) 46 nucleotide (nt) crRNA ("TtCmr-46") or the (C) 40 nt crRNA ("TtCmr-40") incubated with target RNAs (Table 4) with mismatches in the indicated segments.

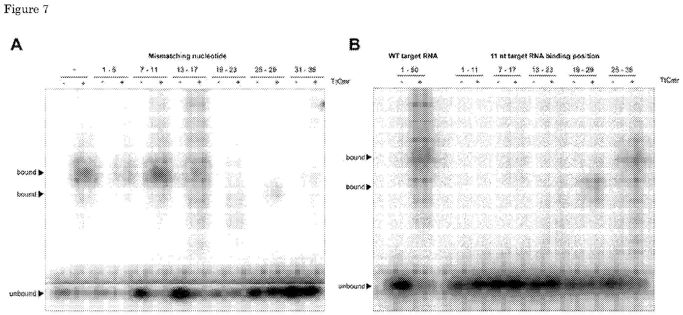

Figure 7. Base pairing of the target RNA with the TtCmr-bound crRNA is initiated at the 3' end of the crRNA. (A) Electrophoretic mobility shift assay (EMSA) analysis of the endogenous TtCmr complex incubated with different target RNAs (Table 4) each containing a stretch of 5 nt mismatching with the TtCmr-bound crRNA. (B) EMSA analysis of the endogenous TtCmr complex incubated with short 11 nt target RNAs (Table 4) complementary to the indicated nucleotides of the TtCmr-bound crRNAs.
Figure 8. A) Absorbance of colourimetric output of detection of Pi was determined for a range of added target RNA concentrations by Cmr as indicated. Reaction time was set at 1h, colour development time at 30 min (direct cOA detection using Malachite Green Assay). B) Absorbance of colorimetric output of detection of target RNA by determination of a level of Pi over time in fold increase (direct cOA detection using Malachite Green Assay). C) Detection of cOA mediated RNAse activity of Csx1 using ssRNA and ssDNA quencher-fluorophore sequences. (indirect cOA detection).
Figure 9. Sensitivity range of *T. thermophilus* Type IIIB Cmr system for native target RNA 4.5. Target recognition was monitored using the indirect cOA visualisation method. A random RNA of similar length was used as a negative off-target control.
Figure 10. : Sensitivity range of *T. thermophilus* Type IIIB Cmr system containing Cmr4 mutant D26N for native target RNA 4.5. Target recognition was monitored using the indirect cOA visualisation method. A random RNA of similar length was used as a negative off-target control.
Figure 11. Comparison between the norovirus target RNA limit of detection for wild-type Cmr complex and and dCmr complex. Target recognition was monitored using the indirect cOA visualisation method. Raw signal output of this assay is presented. A random RNA of similar length was used as a negative off-target control.
Figure 12. cOA production by Type-IIIA CRISPR/Cas complex after addition of complementary target RNA.
Figure 13. Results from the one-pot SARS-CoV-19 N-Gene detection assay. The graph shows data from the "signal production incubation" at 65 °C.
Figure 14. Results from the one-pot SARS-CoV-19 N-Gene detection assay on mink samples. The graph shows data from the "signal production incubation" at 65 °C.

### 4. DETAILED DESCRIPTION OF THE INVENTION

### 4.1 Definitions

The term "Clustered Regularly Interspaced Short Palindromic Repeats (CRISPR)", as is used herein, refers to one or more specialized regions of DNA in the genome of prokaryotic microorganisms. These regions are characterized by the presence of nucleotide repeats that are interspersed by spacer sequences, typically ~25- to ~38 bp direct DNA repeats, separated by unique spacer sequences of a similar length (Grissa et al.,2007), which are derived from previous encounters with invading elements. They serve as a memory to quickly attack these invaders upon a next infection. The genomic region includes one or more CRISPR-associated effector protein (Cas)-encoding genes that are located in the vicinity of the CRISPR loci.

The term "CRISPR crRNA", as is used herein, refers to a CRISPR-derived RNA molecule comprising a spacer sequence and 5 and 3 repeat-derived termini. Said CRISPR crRNA preferably has a length of at least 30 nucleotides, more preferred at least 34 nucleotides, more preferred at least 40 nucleotides, more preferred at least 46 nucleotides. Said CRISPR crRNA preferably is less than 1000 nucleotides, preferably less than 200 nucleotides, preferably less than 100 nucleotides. Said RNA molecule may include ribonucleic acid nucleotide analogues such as inosine, uridine, xanthine, hypoxanthine, 2,6-diaminopurine, and 6,8-diaminopurine-based ribonucleotides and desoxyribonucleotides.

The term "CRISPR-associated effector protein (Cas)", as is used herein, refers to a protein that is associated with CRISPR crRNA. CRISPR/Cas systems are presently grouped into two classes. Class I systems utilize multisubunit Cas complexes, whereas Class II systems use only a single Cas protein to mediate its activity. Class I, type III CRISPR-Cas systems have evolved to target especially RNA sequences. Unique proteins in these systems are Cas3 in Class I systems, Cas9 in Class II systems, and Cas10 in Class I, type III systems.

The term "effector complex", as is used herein, refers to a CRISPR-Cas ribonucleoprotein complex that has nuclease activity and may cleave and inactivate an invading nucleic acid sequence that comprises complementary sequences to the spacer sequence in the CRISPR crRNA.

The term "cyclic oligoadenylate (cOA)", as is used herein, refers to a ring structure comprising 3-6 molecules of Adenosine Mono Phosphate (AMP). The formation of cOA is catalysed by the cyclase domain of Cas10, which is part of type III effector systems.

The term "Type III Cas", as is used herein, refers to a RNA-targeting, multiple subunit CRISPR-associated complex that comprises at least a Cas10 protein.

The term "Type IIIA Cas", as is used herein, refers to a RNA-targeting Type 3 CRISPR/Cas complex that has unspecific DNase activity upon binding to a target RNA molecule. Type IIIA Cas include, for example, Type IIIA Csm complexes from *Staphylococcus thermophilus, Thermus thermophilus* and *Staphylococcus epidermis.*

The term "Type IIIB Cas", as is used herein, refers to a RNA-targeting Type 3 CRISPR-Cas complex that lacks unspecific DNase activity. Said Type IIIB Cas complex is composed of six to seven proteins. Type IIIB Cas include, for example, Type IIIB Cmr complexes from *Pyrococcus furiosus, Thermus thermophilus* and *Sulfolobus solfataricus.*

The term "PPi" or phosphonato phosphate, as is used herein, refers to a salt or ester of pyrophosphoric acid. Alternative names are pyrophosphate, diphosphate and dipolyphosphate.

The term "inorganic pyrophosphatase", or inorganic diphosphatase, as is used herein, refers to an enzyme that catalyzes the conversion of one ion of pyrophosphate to two phosphate ions. The enzyme is of the enzyme class EC 3.6.1.1.

The term "cOA-dependent, non-specific effector endoribonuclease", as is used herein, refers to ribonucleases which degrade RNA non-specifically using a HEPN (Higher Eukaryotes and Prokaryotes, Nucleotide binding) active site. These nucleases get activated by binding of a cOA messenger using their CRISPR-associated Rossmann fold (CARF) domain. Examples of such non-specific effector endoribonuclease are the Cas accessory proteins Csx1 and Csm6.

The term "biosensor", or "biological sensor", as is used herein, refers to a sensing device comprising a CRISPR-based ribonucleic acid system according to the invention. A signal that is generated when the CRISPR-based ribonucleic acid system interacts with a RNA molecule that is complementary to the crRNA, for example a colorimetric, fluorometric, fluorescent or bioluminescent signal, may be coupled to a transducer, allowing quantification of the signal. The signal is preferably converted by means of a suitable transducer into a measurable electrical parameter such as a current or voltage.

### 4.2 CRISPR/Cas-based ribonucleic acid detection system

Provided herein is a clustered regularly interspaced short palindromic repeats (CRISPR) based ribonucleic acid (RNA) detection system. Said system comprises an effector complex comprising a Type III CRISPR-associated effector protein (Cas) and at least one CRISPR RNA (crRNA) that binds to a target nucleic acid molecule and methods and means for directly or indirectly determining a level of cyclic oligoadenylate (cOA).

Said CRISPR/Cas-based ribonucleic acid detection system preferably is derived from a thermophilic organism such as *Pyrococcus furiosus, Sulfolobus solfataricus* or *Thermus thermophilus.* An advantage of a CRISPR/Cas-based ribonucleic acid detection system from a thermophilic organism is that detection can be performed at an elevated temperature, for example between 40 °C and 80 °C, preferably between 50 °C and 70 °C, such as between 55 °C and 65 °C, preferably about 65 °C. Incubation at this temperature may accelerate the cOA synthesis reaction, when compared to incubation at a lower temperature. In addition, said elevated temperature may inactivate a nuclease, such as a DNase or RNase, or protease that is present in the sample.

In addition, an advantage of a CRISPR/Cas-based ribonucleic acid detection system from a thermophilic organism may provide an increased stability to the system such as it may be stored for a longer period of time, when compared to a CRISPR/Cas-based ribonucleic acid detection system from a mesophilic organism.

Said ribonucleic acid detection system is based upon the prokaryotic CRISPR/Cas system that constitutes an acquired immunity system to protect prokaryotic cells against invading viruses and plasmids, analogous to the eukaryotic RNA interference (RNAi) system ( Makarova et al., 2006. Biol Direct 1: 1-7). A CRISPR-Cas immune response consists of three distinct stages: 1) Adaptation, by which a part of the target DNA of an invader is excised and inserted into a CRISPR array; 2) Expression and maturation of CRISPR (cr) RNA and association of a CRISPR/Cas complex; and 3) Interference when the crRNA is used as the guide to recognize sequences complementary to the matured CRISPR sequence in an invading genome of a virus or plasmid, followed by cleavage and inactivation of the foreign nucleic acid by a Cas nuclease.

A general structure of Type III CRISPR/Cas complexes is that they comprise multiple subunits of Cas7 and Cas11 (Staals et al., 2013. Mol. Cell 52: 135-145; Staals et al., 2014. Mol. Cell 56: 518-530), which are capped off at one side by Cas5 and Cas10. Of these, Cas7 provides RNase activity upon recognition of target RNA by the pre-loaded RNA guide.

It has been shown that target recognition promotes the production of cyclic oligoadenylates (cOA) by the Cas10 Palm domain (Kazlauskiene et al., 2017. Science 357: 605-609; Niewoehner et al., 2017. Nature 548: 543-548). Although cOA species are known signaling molecules in eukaryotes, no such activity had been reported before in prokaryotic hosts. However, research indicates that the presence of cOA leads to a large increase in RNase activity by Csm6 or Csx1 family members. These proteins are often encoded in the Type III loci, but do not associate directly with the ribonucleoprotein complex. Instead, recognition of invader RNA transcripts may result in targeted RNA degradation by Cas7, non-specific DNA degradation by a Class I, type IIIA Cas10, as well as cOA production leading to the activation of Csm6 or Csx1, thereby causing collateral cleavage of other nearby single stranded RNA molecules.

Methods of directly determining a level of cOA preferably include methods to determine a level of pyrophosphate or PPi. The formation of pyrophosphate is coupled to the formation of cOA from ATP by a CRISPR/Cas associated protein such as Cas10. The formation of cOA, consisting of 3-6 AMP units, results in the simultaneous formation of 3-6 molecules of PPi.

As an alternative, the detection system according to the invention may comprise an inorganic pyrophosphatase, resulting in the breakdown of PPi and the formation of two inorganic phosphate molecules for each PPi molecule. Hence, by determining a level of inorganic phosphate, the number of detectable molecules is amplified from 1 molecule of cOA to 6-12 molecules of Pi.

A preferred inorganic pyrophosphatase is an enzyme that is active at the same or similar temperature as the Type III CRISPR/Cas-based RNA detection system. In addition, it is preferred that the inorganic pyrophosphatase is active under the same or similar conditions as the Type III CRISPR/Cas-based RNA detection system, including at the same or similar pH and at the same or similar salt concentrations. For example, if the Type III CRISPR/Cas-based RNA detection system has an optimal activity at 50 °C, it is preferred that the inorganic pyrophosphatase is active at this temperature. Preferably, the activity of the inorganic pyrophosphatase at 50 °C is such that essentially all PPi molecules are broken down into inorganic phosphate molecules. Said break down preferably is instantaneous. Similarly, the activity of the inorganic pyrophosphatase at the chosen pH and salt concentrations is such that essentially all PPi molecules are broken down into inorganic phosphate molecules. Said break down preferably is instantaneous.

A preferred inorganic pyrophosphatase is from a thermophylic organism such as *Pyrococcus furiosus, Sulfolobus solfataricus* and *Thermus thermophilus,* allowing simultaneous, isothermal detection. In this way, a level of cOA can be directly determined by determining a level of Pi.

PPi and Pi can be detected using methods known in the art, including colorimetric-, fluorometric-, fluorescent- or bioluminescent-based assays.

Suitable methods for determining a level of PPi include a fluorometric and/or colorimetric pyrophosphate (PPi) Assay Kit (Biovision Inc., Milpitas, CA); a fluorescent EnzChek^{®} Pyrophosphate Detection Kit (ThermoFisher Scientific; Waltham, MA); a fluorometric Pyrophosphate Assay Kit (SigmaAldrich, Saint Louis, MO); and a luminescent PPiLight^{™} assay (Lonza Group A.G., Bazel, Switzerland).

Suitable methods for determining a level of inorganic phosphate or Pi include a colorimetric PiColorLock^{™} assay (Expedeon, Cambridge, UK); a colorimetric Malachite Green Phosphate Assay Kit (SigmaAldrich, Saint Louis, MO); a fluorescent Phosphate Sensor (ThermoFisher Scientific; Waltham, MA); a luminescent readout following conversion of ADP to ATP (US patent application US20140273036A); a fluorescent chemosensor (Meng et al., 2015. RSC Advances 5: 53189-53197); and photoluminescent graphene quantum dots combined with Europium ions (Bai et al., 2013. Chemistry 19: 3822-3826).

The methods and means for directly determining a level of cyclic oligoadenylate (cOA) preferably include at least one substrate and, if required, an enzyme that allows at least one of the indicated detection methods for PPi or Pi.

A preferred method is a colorimetric method such as the Malachite Green Phosphate Assay Kit, allowing fast determination of a level of PPi or Pi, as a direct determination of a level of cOA.

Methods of indirectly determining a level of cOA preferably include methods to determine an activity of a cOA-dependent, non-specific effector nuclease such as CRISPR ancillary nuclease 1 (Can1) or Can2 and, preferably, methods to determine an activity of cOA-dependent, non-specific effector endoribonuclease such as Csx1. For this, a detection system according to any the invention preferably includes a cOA-dependent, non-specific effector endoribonuclease such as Csx1.

Said cOA-dependent, non-specific effector nuclease, preferably endoribonuclease, preferably is an enzyme that is active at the same or similar temperature as the Type III CRISPR/Cas-based RNA detection system. In addition, it is preferred that the cOA-dependent, non-specific effector endoribonuclease is active under the same or similar conditions as the Type III CRISPR/Cas-based RNA detection system, including at the same or similar pH and at the same or similar salt concentrations. For example, if the Type III CRISPR/Cas-based RNA detection system has an optimal activity at 65 °C, it is preferred that the cOA-dependent, non-specific effector endoribonuclease is active at this temperature. Preferably, the activity of the cOA-dependent, non-specific effector endoribonuclease at 65 °C is such that the cOA-induced activity of the cOA-dependent, non-specific effector endoribonuclease results in the production of detectable amounts of a reaction product of the substrate of said cOA-dependent, non-specific effector endoribonuclease. Similarly, the activity of the cOA-dependent, non-specific effector endoribonuclease at the chosen pH and salt concentrations is such that cOA-induced activity of the cOA-dependent, non-specific effector endoribonuclease results in the production of detectable amounts of a reaction product of the substrate of said cOA-dependent, non-specific effector endoribonuclease.

A substrate for the cOA-dependent, non-specific effector endoribonuclease preferably is a RNA molecule, cleavage of which can be detected. Detection may be performed by any method known in the art. For example, detection may be performed directly by mass spectrometry, for example ultra-high performance liquid chromatography (UHPLC) coupled to tandem mass spectrometry (LC-MS/MS) in positive electrospray ionization mode. The LC-MS/MS analysis may be performed, for example by using a high end UHPLC chromatographic system coupled to a triple-quadrupole mass-spectrometer.

Detection may further be performed by liquid-liquid phase separation (LLPS; Spoelstra et al., 2018. BioRXiv, CSHL (doi.org/10.1101/471482).

A preferred substrate for the cOA-dependent, non-specific effector endoribonuclease is a RNA molecule that is tagged with a fluorescent reporter molecule on one end and a quencher on the other end. The close proximity of the reporter to the quencher prevents detection of its fluorescence. Cleavage of the substrate by activation of the cOA-dependent, non-specific effector endoribonuclease breaks the reporter-quencher proximity and thus allows unquenched emission of fluorescence, which can be detected after excitation with a laser. An increase in the activity of the cOA-dependent, non-specific effector endoribonuclease therefore causes a proportional increase in fluorescence due to the cleavage of the substrate and removal of the quencher that quenches the fluorescent reporter.

Separate from activation of the non-specific effector endoribonuclease, or in addition thereto, determination of a level of target recognition may also be performed by determination of activation of the non-specific effector DNase activity that is present in Type IIIA CRISPR/Cas effector complexes using an appropriate substrate for said nuclease. Said appropriate substrate preferably is a DNA molecule that is tagged with a fluorescent reporter molecule on one end and a quencher on the other end. The close proximity of the reporter to the quencher prevents detection of its fluorescence. Cleavage of the substrate by activation of the cOA-dependent, non-specific effector DNase breaks the reporter-quencher proximity and thus allows unquenched emission of fluorescence, which can be detected after excitation with a laser. An increase in the activity of the cOA-dependent, non-specific effector DNase therefore causes a proportional increase in fluorescence due to the cleavage of the substrate and removal of the quencher that quenches the fluorescent reporter. The use of both the activation of the non-specific effector endoribonuclease and the activation of the non-specific effector DNase allows determination of presence or absence of two independent target RNA molecules in one single assay, provided that two independent ribonucleoprotein complexes are used, one of which specifically activates the non-specific effector DNase while the other specifically allows indirect or direct determination of cOA levels. A person skilled in the art will understand that for this, the fluorescent labels that are present on substrate by activation of the cOA-dependent, non-specific effector endoribonuclease and the non-specific effector DNase must be sufficiently different to allow determination of a level of each activity as a measure for determining a level of cOA.

Preferred fluorescent labels may be selected from Atto425 (ATTO-TEC GmbH, Siegen, Germany), Atto 647N (ATTO-TEC GmbH, Siegen, Germany), YakimaYellow (Epoch Biosciences Inc, Bothell, WA, USA), Cal610 (BioSearch Technologies, Petaluma, CA, USA), Cal635 (BioSearch Technologies, Petaluma, CA, USA), FAM (Thermo Fisher Scientific Inc., Waltham, MA USA), TET (Thermo Fisher Scientific Inc., Waltham, MA USA), HEX ((Thermo Fisher Scientific Inc., Waltham, MA USA), cyanine dyes such as Cy5, Cy5.5, Cy3, Cy3.5, Cy7 (Thermo Fisher Scientific Inc., Waltham, MA USA), Alexa dyes (Thermo Fisher Scientific Inc., Waltham, MA USA), Tamra (Thermo Fisher Scientific Inc., Waltham, MA USA), ROX (Thermo Fisher Scientific Inc., Waltham, MA USA), JOE (Thermo Fisher Scientific Inc., Waltham, MA USA), fluorescein isothiocyanate (FITC, Thermo Fisher Scientific Inc., Waltham, MA USA), Yakima Yellow^{®} (YY; Epoch Biosciences, Bothell, Washington) and tetramethylrhodamine (TRITC, Thermo Fisher Scientific Inc., Waltham, MA USA). Said substrate is preferably labeled at the 5' end with a detectable label, preferably a fluorescent label.

Quenchers, for example tetramethylrhodamine TAMRA, dihydrocyclopyrroloindole tripeptide minor groove binder, are known in the art. Preferred quenchers include Black Hole Quencher^{®}-1 (BHQ1) and BHQ2 (Biosearch Technologies, Petaluma, CA, USA). The BHQ1 dark quencher has strong absorption from 480 nm to 580 nm, which provides quenching of fluorophores that fluoresce in this range, such as FAM, TET, CAL Fluor^{®} Gold 540, JOE, HEX, CAL Fluor Orange 560, and Quasar^{®} 570 dyes. The BHQ2 dark quencher has strong absorption from 599 nm to 670 nm, which provides quenching of fluorophores that fluoresce in this range, such as Quasar^{®} 570, TAMRA, CAL Fluor^{®} Red 590, CAL Fluor Red 610, ROX, CAL Fluor Red 635, Pulsar^{®} 650, Quasar 670 and Quasar 705 dyes. BHQ1 and BHQ2 may quench fluorescence by both FRET and static quenching mechanisms.

A suitable commercial substrate is provided by the RNaseAlert^{®} Lab Test Kit v2 (ThermoFisher Scientific; Waltham, MA).

A preferred cOA-dependent, non-specific effector endoribonuclease is from a thermophylic organism such as *Pyrococcus furiosus, Sulfolobus solfataricus* and *Thermus thermophilus,* allowing simultaneous, isothermal detection of the activity of the cOA-dependent, non-specific effector endoribonuclease.

The methods and means for indirectly determining a level of cyclic oligoadenylate (cOA) preferably include a cOA-dependent, non-specific effector endoribonuclease and a substrate of said cOA-dependent, non-specific effector endoribonuclease.

### 4.3 Production of proteins

The ribonucleic acid detection system disclosed herein is based on the *in vitro* assembly of a ribonucleoprotein complex, preferably a Type IIIB complex. The required Cas proteins preferably are from a thermophilic organism such as *Thermus thermophilus.* Said proteins preferably are expressed and purified from a suitable expression system.

Commonly used expression systems for heterologous protein production include E. *coli, Bacillus* spp., baculovirus, yeast, fungi, most preferably filamentous fungi or yeasts such as *Saccharomyces cerevisiae* and *Pichia pastoris,* eukaryotic cells such as Chinese Hamster Ovary cells (CHO), human embryonic kidney (HEK) cells and PER.C6^{®} cells (Thermo Fisher Scientific, MA, USA) and plants. The efficiency of expression of recombinant proteins in heterologous systems depends on many factors, both on the transcriptional level and the translational level.

Cas proteins preferably are produced using prokaryotic cells, preferably E. *coli.* Said Cas proteins are preferably produced by expression cloning of the proteins to a prokaryotic cell of interest, preferably E. *coli.* Said expression construct, preferably DNA, is preferably produced by recombinant technologies, including the use of polymerases, restriction enzymes, and ligases, as is known to a skilled person. Alternatively, said expression construct is provided by artificial gene synthesis, for example by synthesis of partially or completely overlapping oligonucleotides, or by a combination of organic chemistry and recombinant technologies, as is known to the skilled person.

As an alternative, or in addition, Cas proteins may be isolated from a thermophilic organism by expression of a tagged Cas protein in said thermophilic organism, and isolation of ribonucleoprotein complex comprising said Cas proteins on the basis of the tag. Said isolated ribonucleoprotein complexes can be isolated using the tagged Cas protein.

Said expression construct is preferably codon-optimised to enhance expression of the Cas proteins in a prokaryotic cell of interest, preferably *E*. *coli.* Further optimization preferably includes removal of cryptic splice sites, removal of cryptic polyA tails and/or removal of sequences that lead to unfavorable folding of the mRNA. In addition, the expression construct preferably encodes a protein export signal for secretion of the Cas proteins out of the cell into the periplasm of prokaryotes, allowing efficient purification of the Cas proteins.

Methods for purification of Cas proteins are known in the art and are generally based on chromatography such as affinity chromatography and ion exchange chromatography, to remove contaminants. In addition to contaminants, it may also be necessary to remove undesirable derivatives of the product itself such as degradation products and aggregates. Suitable purification process steps are provided in Berthold and Walter, 1994 (Berthold and Walter, 1994. Biologicals 22: 135-150).

As an alternative, or in addition, recombinant Cas proteins may be tagged with one or more specific tags by genetic engineering to allow the protein attach to a column specific to the tag and therefore be isolated from impurities. The purified protein is then exchanged from the affinity column with a decoupling reagent. The method has been increasingly applied for purifying recombinant protein. Conventional tags for proteins, such as histidine tag, are used with an affinity column that specifically captures the tag (e.g., a Ni-IDA column for the histidine tag) to isolate the protein from other impurities. The protein is then exchanged from the column using a decoupling reagent according to the specific tag (e.g., imidazole for histidine tag). This method is more specific, when compared with traditional purification methods.

Suitable further tags include c-myc domain (EQKLISEEDL), hemagglutinin tag (YPYDVPDYA), maltose-binding protein, glutathione-S-transferase, FLAG tag peptide, biotin acceptor peptide, streptavidin-binding peptide and calmodulin-binding peptide, as presented in Chatterjee, 2006 (Chatterjee, 2006. Cur Opin Biotech 17, 353-358). Methods for employing these tags are known in the art and may be used for purifying Cas proteins.

Methods for expression proteins in *E. coli* are known in the art and can be used for expression and purification of the Cas-proteins.

In a preferred method, Cas proteins are expressed in *E. coli* from a codon-optimized expression construct. Said construct is placed in a bicistronic expression plasmid containing a Strep-tag and amino-acid sequence Glu-Asn-Leu-Tyr-Phe-Gln-(Gly/Ser) at the N-terminus, which amino acid sequence is recognized by a Tobacco Etch Virus (TEV) protease. The expression plasmid is transformed into E. *coli,* for example in strain Bl21(DE3). Following growth at 37 °C in a desired culture volume, until OD600 of ~0.6, the culture is placed on ice for 1 hour after which isopropyl β-D-1-thiogalactopyranoside is added to a final concentration of 0.1 mM. The culture is then incubated at 18 °C for ~16 hours (overnight). The cells are harvested and lysed in Buffer A (100 mM Tris-HCl, 150 mM NaCl) by sonication and subsequently spun down at 30.000g for 45 min. The clarified lysate in filtered and run over a pre-equilibrated StrepTrap FPLC column (GE Healthcare, Chicago, IL). After washing the column with Buffer A until no more protein is present in the flow through, the protein of interest is eluted using Buffer B (100 mM Tris-HCl, 150 mM NaCl & 2.5 mM D-desthiobiotin). The protein is cleaved from the affinity tag by addition of TEV protease and left to incubate overnight at 4 °C. The protein of interest is separated from the mixture by a HisTrap and StrepTrap affinity chromatography step, from which the flow through is collected. If required, an additional size exclusion chromatography is added to achieve higher purity.

Preferred Cas proteins comprise Csm or Cmr proteins, at least Cmr 1 and Cmr 4, preferably Cmr 1-6, or at least Csm 2 and Cas10 (Csm1), preferably Csm 2-5 and Cas 10, more preferably Csm 1-6.

Preferred proteins include proteins having at least 80 % sequence identity with the amino acid sequences as referred in Table 2, preferably at least 90% sequence identity, preferably are proteins with amino acid sequences as referred to in Table 2. However, mutants of said proteins, including insertion mutants, deletion mutants, chimeric proteins and amino acid substituted proteins may also be used in a CRISPR-based ribonucleic acid system according to the disclosure.

To increase sensitivity of the Type III CRISPR/Cas detection method, catalytically dead mutant Cmr and/or Csm complexes are preferably created and used in a detection system of the disclosure. The term "catalytically dead" refers to the target RNA-digesting activity of the CRISPR-based ribonucleic acid system according to the disclosure. These mutants are referred to as dCmr and dCsm. The mutations are introduced in the Cmr4 and Csm3 subunits that are responsible for target binding and cleaving and are selected for abolishment of target cleavage, while maintaining target binding. A number of mutations in the Cmr4 protein (H15A,D26A,E277A), have been described, with the strongest catalytic impairment being observed in Cmr4 D26A and D26N mutations (Benda et al., 2014. Molecular Cell 56: 43-54; Ramia et al., 2014. Cell Reports 9: 1610-1617). This inactivation mutation is experimentally proven to work in *Pyrococcus furiosis,* and sequence alignments of Cmr4 orthologs shows that this amino acid is highly conserved (data not shown). In addition, crystallography data shows that this particular amino acid is located in a groove of the complex where target RNA is expected to bind (data not shown). Together, these results indicate that the D26 amino acid residue is important for the catalytic activity of Cmr4. Alteration of this residue will result in a *Thermus thermophilus* dCmr4 mutant. Next to this, alignment of the Pf_Cmr4 amino acid sequence with orthologs of Csm3 also show that D26 is one of the few amino acids that is conserved between the two Type III systems and could therefore also be used to create a dCsm3 mutant. Further cleavage-dead mutations include a E227A and E228 double mutant of Cmr4, and Cmr4 D86A (Ramia et al., 2014. Cell Reports 9: 1610-1617; Zhu and Ye, 2015. Nucleic Acids Res 43: 1257-1267). In addition, a Csm3 D32A is a good candidate for a cleavage-dead Type IIIA Csm mutant (Samai et al., 2015. Cell 161: 1164-1174).

Additionally, Jia et al. and Park et al. describe a dCsm3 mutant created by the D36A substitution (Jia et al., 2019. Mol Cell 73: 264-277; Park et al., 2017. EMBO reports 18: 826-840). A double mutant, K56A and R60A, was also mentioned, but did not fully abolish target cleavage in Csm3.

An additional type of mutation is based on a damage control by the natural CRISPR/Cas -based defense system. After binding of cOA in a CRISPR-associated Rossmann fold (CARF) domain of Csx1/Csm6, its Higher Eukaryotes and Prokaryotes Nucleotide-binding (HEPN) domain is activated and the protein will indiscriminately start to cleave RNA (Kazlauskiene et al., 2017. Science 357: 605-609). In the normal biological setting, cOA is degraded by Csx1/Cms6 itself to avoid sustained activation and therefore sustained RNase activity (Athukoralage et al., 2019. J Mol Biol 431: 2894-2899, Garcia-Doval et al., 2020. Nature Communications 11: 1-9). Recently, the mechanism of this cOA degradation was elucidated by making use of mutant versions of the Csx1/Csm6 protein. It was shown that a T10A, T10A/T11A or T11A Csx1/Csm6 mutant was not able to degrade the cOA and resulted in prolonged RNase activity (Athukoralage et al., 2019. J Mol Biol 431: 2894-2899; Garcia-Doval et al., 2020. Nature Communications 11: 1-9). This prolonged RNase activity by Csx1/Csm6, for example by employing a T10A, T10A/T11A or T11A Csx1/Csm6 mutant or equivalent thereof, can be exploited for a more sensitive and rapid readout for diagnostic purposes.

### 4.4 Assembly of ribonucleoprotein complexes

Although differences exist between different Type III subtypes (Cmr, Csm), a general structure consisting of multiple subunits of Cas7 and Cas11 (Staals et al., 2013. Mol. Cell 52: 135-145; Staals et al., 2014. Mol. Cell 56: 518-530), which are capped off at one side by Cas5 and Cas10. Of these, Cas7 provides RNase activity upon recognition of target RNA by the pre-loaded RNA guide. Furthermore, this recognition also results in DNase activity by the Cas10 HD domain (Kazlauskiene et al., 2016. Mol Cell 62: 295-306).

Type III CRISPR/Cas systems can be differentiated further into various subtypes, including Type IIIA Csm and Type IIIB Cmr (Staals et al., 2013, 2014. *Ibid*). The effector complex of *Thermus thermophilus* Cmr (ttCmr46) consists of 12 subunits with the stoichiometry of Cmr1₁2₁3₁4₄5₃6₁ (Staals et al., 2014. *Ibid*; Taylor et al., 2015. Science 348: 581-586) and a 46 nt crRNA. Novel research by Staals et al. has revealed a similar complex with a 40 nt crRNA (Table 1).

**Table 1. Subunit composition of TtCmr40 and 46**

| **Cmr-46** | **Cmr-40** | |
|---|---|---|
| 1x | 1x | Cmr1 (Cas7-like) |
| 1x | 1x | Cmr2 (Cas10, LS) |
| 1x | 1x | Cmr3 (Cas5) |
| 4x | 3x | Cmr4 (Cas7) |
| 3x | 2x | Cmr5 (SS) |
| 1x | 1x | Cmr6 (Cas7-like) |

Type IIIA Csm complexes are composed in a similar fashion as Cmr (Csm1₁2₃3₅4₁5₁) (Tamulaitis et al., 2017. Trends Microbiol 25: 49-61). This complex can be assembled by adding all subunits in the correct molar ratios and leaving the reaction mixture to incubate for a period of time, for example 30 min, at the incubation temperature, for example 60 °C or 65 °C.

Purified Cas proteins, such as Csm or Cmr proteins, are assembled onto a suitable crRNA. In one embodiment, said crRNA and the assembled ribonucleoprotein complex are present in an aqueous solution. If required for subsequent determination of a level of cOA, one of the Cas proteins, for example a CAP Cas such as Cmr 6, may be tagged, for example with a histidine tag and/or a strep tag, and attached to a surface, preferably at a defined position on a surface Said surface may be a solid surface, such as glass, plastic or silicon. Said surface may be present in a receptacle such as a cup, for example an Eppendorf tube, or in a well of a microplate, or in a device.

Recent research by Mogila et al. has revealed the roles of the individual subunits of *Streptococcus thermophilus* Csm (StCsm) (Mogila et al., 2019. Cell Reports 26: 2753-2765). Additionally, a minimal Csm complex only containing Csm3, Csm4 and Cas10 (Cmr1) subunits (and crRNA) was designed, which still retained all three catalytic activities (RNase, ssDNase, cOA synthase).

Abolishing several subunits while still retaining all catalytic activities holds promise for integration in practical use of Type III CRISPR/Cas systems. Most notably, cOA production was not reported to be affected (Mogila et al., 2019. *Ibid*).

These minimal complexes would include, as described by Mogila et al., Cmr1, 3 and 4 for Type IIIA CRISPR/Cas, and Cmr 2, 3 and 4 for Type IIIB CRISPR/Cas, with variation of copy numbers of these subunits being possible. To increase cOA production, and specificity and sensitivity of target detection the number of Csm3/Cmr4 subunits may be increased.

Additionally, these subunits may be mutated to optimize their activity.

Said Cmr proteins preferably are provided to a suitable crRNA with a stoichiometry of Cmr1:Cmr3:Cmr4:crRNA of 1:1:4:1, and of Cmr1:2:3:4:5:6:crRNA of 1:1:1:4:3:1:1.

A nucleoprotein complex can be assembled on a TtCmr46 crRNA as follows:
First, 3.5 µL crRNA (700 ng) was added to 3.5 µL 1X Cmr buffer (20 mM Tris-HCl pH 8.0, 150 mM NaCl). Subsequently, the subunits were added to the reaction mixture in a specific order (Cmr3, Cmr2, Cmr4, Cmr5, Cmr6, Cmr1) to a final concentration of .5 µM, 2.5 µM, 10 µM, 7.5 µM, 2.5 µM and 2.5 µM, respectively, to make up a total reaction volume of 20 µL. The reaction mixture may be incubated at 65°C for 30 minutes.

A preferred method for reconstituting a Class I, Type 3A complex comprises addition of 3.5 µL crRNA (700 ng) to 3.5 µL 1X Cmr buffer (20 mM Tris-HCl pH 8.0, 150 mM NaCl), followed by the addition of the subunits in the order Csm4, Csm1, Csm3, Csm2, Csm5 to a final concentration of 2.5 µM, 2.5 µM, 12.5 µM, 7.5 µM and 2.5 µM, respectively, to make up a total reaction volume of 20 µL. The reaction mixture may be incubated at 60°C or 65 °C for 30 minutes.

**Table 2. Preferred Cmr and Csm sequences**

| **Subunit name** | **Cas name** | ***T. thermophilus* Locus tag** | **Uniprot identifier** | **GeneID identifier** |
|---|---|---|---|---|
| **Cmr1** | Cas7-like | tthb162 | Q53W07 | 3169605 |
| **Cmr2** | Cas10 | tthb160 | Q53W09 | 3169601 |
| **Cmr3** | Cas5 | tthb161 | Q53W08 | 3169600 |
| **Cmr4** | Cas7 | tthb163 | Q53W06 | 3168402 |
| **Cmr5** | Cas11 | tthb164 | Q53W05 | 3169607 |
| **Cmr6** | Cas7-like | tthb165 | Q53W04 | 3168401 |

| **Subunit name** | **Cas name** | ***T. thermophilus* Locus tag** | **Uniprot identifier** | **GeneID identifier** |
|---|---|---|---|---|
| **Csm1** | Cas10 | tthb147 | Q53W19 | 3169523 |
| **Csm2** | Cas11 | tthb148 | Q53WF6 | 3169530 |
| **Csm3** | Cas7 | tthb149 | Q53WF5 | 3169534 |
| **Csm4** | Cas5 | tthb150 | Q53WF4 | 3169524 |
| **Csm5** | Cas7-like | tthb151 | Q53W18 | 3169228 |
| **Csm6** | Csx1 | tthb144 | Q53W22 | 3169588 |
| **Csm6** | Csx1 | tthb152 | Q53W17 | 3169219 |
| **Csm6** | Csx1 | tthb155 | Q53W14 | 3169545 |

| | | | | |
|---|---|---|---|---|
| GeneID identifiers are from the gene database of NCBI RefSeq genomes | | | | |

### 4.5 Methods of detecting RNA

Conventional CRISPR/Cas systems that are being used for diagnostic purposes, e.g. Cas12/Cas14 (UC Berkeley, CA, USA) and Cas13-based systems (Broad Institute, MA, USA), are dependent on the presence of a specific motif adjacent to the target site. This mechanism, in the biological setting, is used to discriminate self from non-self. Without this motif these Cas proteins are not able to cleave their target and their use a diagnostic system is not possible. For Cas12 and Cas13 these motifs are called a protospacer adjacent motif (PAM) and protospacer flanking site (PFS), respectively [Westra et al., 2013. PLoS Genet 9: e1003742; Abudayyeh et al., 2016. Science 353: aaf5573]. In the case of some Cas12/Cas14 proteins this required PAM is TTTN/TTTA, greatly limiting the choice in target sequences. For Cas13 the required motif is limiting to a lesser extent, favoring an H nucleotide (A, C, U) adjacent to the target sequence.

The Type-III(B) CRISPR systems employ a different self versus non-self mechanism which prevents self-immunity by checking complementarity between the 5'-repeat tag of crRNA and the corresponding 3'-protospacer flanking sequence [Guo et al., 2019. RNA Biol 16: 1513-1520]. Complementarity between these two regions affects the relative cOA production to a certain degree but there is no requirement of a certain PAM/PFS-like sequence in order for the Type-III to function as desired, giving a great advantage over currently used systems [Guo et al., 2019. RNA Biol 16: 1513-1520].

Moreover, the use of Cas13 target selection is also limited by potential secondary structures that are formed in the target RNA [Smargon et al., 2017. Mol Cell 65: 618-630]. Diagnostic applications that are currently being used operate at 37 °C, in contrast to the Type-IIIB system for *Thermus thermophilus* which operates at 65 °C [Staals et al., 2013. Mol Cell, 52: 135-145]. This elevated temperature reduces the amount of secondary RNA structures potentially forming in target RNA sequences [Wan et al., 2012. Mol Cell 48: 169-181].

The methods of the invention for detecting specific RNA sequences may be used in human healthcare, veterinary diagnostics, detection of plant pathogens, detection of water contaminants and the detection of food and feed contaminants. In addition, the methods of the invention for detecting specific RNA sequences may be used for detecting beneficial organisms. In general, the methods of the invention can be used for detection of bacterial, fungal, archaeal, protest, protozoal, eukaryotic, viral and viroidal pathogens. In addition, the methods of the invention may also be used for detection and diagnosis of genetic alterations or traits that are expressed as RNA molecules, in human, animals and plants.

For human healthcare and veterinary diagnostics, nucleic acid material, including RNA, is preferably isolated from a biological fluid, preferably from cerebrospinal fluid, saliva, nasopharyngeal secretion, oropharyngeal secretion, sweat, urine stool, or blood. The term "blood" includes blood plasma, which is prepared by removing red and white blood cells, for example by centrifugation, and blood serum, which is prepared by formation of a blood clot, and removal of the clot using, for example, a centrifuge. A preferred biological fluid is blood. Methods and compositions for isolation of nucleic acid material from biological fluids, particularly blood, preferably employ aqueous solvents without use of organic solvents and chaotropic salts.

If necessary, nucleic acid material, including RNA, may be purified from a sample using, for instance, a combination of physical and chemical methods. Commercially available systems for nucleic acid isolation are preferably used, such as the NucliSENS^{®} easyMAG^{®} or NucliSENS^{®} miniMAG^{®} nucleic acid extraction system (bioMérieux, Marcy l'Etoile, France), or a MagNA Pure 96 System (Roche Diagnostics, Almere, The Netherlands).

For this, RNA may be isolated from a sample by any technique known in the art, including but not limited to suitable commercial RNA isolation kits include Trizol (Invitrogen; Carlsbad, California), RNAqueous^{®} (Applied Biosystems/Ambion, Austin, Tx), Qiazol^{®} (Qiagen, Hilden, Germany), Agilent Total RNA Isolation Lits (Agilent; Santa Clara, California), RNA-Bee^{®} (Tel-Test. Friendswood, Texas), the RNeasy mini kit (Qiagen, Venlo, The Netherlands), and Maxwell^{™} 16 Total RNA Purification Kit (Promega; Madison, Wisconsin). The isolated RNA, preferably mRNA, is preferably reverse transcribed with the aid of a RNA-dependent DNA polymerase into single or double stranded cDNA.

Use of Type III CRISPR/Cas according to the disclosure for diagnostic purposes include, but is not limited to, medical diagnostics such as detection of urinary tract infection, respiratory tract infection especially of SARS-CoV-2 and respiratory syncytial virus (RSV), blood infection (sepsis), expression of antibiotic resistance markers such as methicillin-resistant *Staphylococcus aureus* markers and extended spectrum beta-lactamases markers, gastrointestinal infection, skin infection, odontogenic infection, vaginal infection such as *Candidiasis, Trichomonas vaginalis,* and *Gardnerella,* male reproductive system infection, tropical infectious disease such as malaria, trypanosoma, dengue fever, Zika fever, chickungunya fever, detection of sexually transmissible diseases caused by Chlamydia spp., gonorrhea, human immunodeficiency virus, Herpes spp., syphilis, and of genetic defects including cancer and autoimmune disease.

The Type III CRISPR/Cas according to the invention may further be used for veterinary diagnostics including detection of cattle infectious diseases such as mastitis, bluetongue, foot and mouth disease, *Salmonella* spp., *Klebsiella* spp., *Campylobacter* spp., pig infectious diseases such as respiratory diseases, dermatitis, diarrhea, and infections by porcine parvovirus; sheep and goat infectious diseases such as Clostridial diseases, soremouth, pneumonia and infections with Rift Valley Diseae Virus; poultry infectious diseases such as infectious bronchitis, Salmonella spp.; feline infectious diseases such as infections with feline immunodeficiency virus (FIV) and feline leukaemia virus (FeLV), respiratory infections; canine infectious diseases such as rabies, and infections with *Bordetella, Leptospira,* and *Boriella.*

The Type III CRISPR/Cas detection system according to the invention may further be used for detection of plant pathogens such as detection of certain fungi such as *Ascomycetes* species and *Basidiomycetes* species, certain fungi-like organisms such as oomycetes and phytomyxea, certain bacteria such as *Burkholderia,* proteobacteria, and Pseudomonas species; viruses, viroids and virus-like organisms such as tobacco mosaic virus, cauliflower mosaic virus; nematodes such as *Meloidogyne chitwoodi* and *M. fallax*; and protozoa and algae such as *Phytomonas* and *Cephaleuro.*

The Type III CRISPR/Cas detection system according to the invention may further be used for detection of water contaminants, including detection of bacterial contamination such as *Vibrio cholerae, E. coli, Shigella* spp., *Legionella* spp., *Salmonella* spp.; viral contamination such as hepatitis A, hepatitis E, poliovirus; algae contamination such as presence of *Desmodesmus* spp; and parasitic contamination such as presence of *Dracunculiasis* spp.

The Type III CRISPR/Cas detection system according to the invention may further be used for detection of food and feed contaminants including bacterial contamination such as presence of *Clostridium botulinum, E. coli, Listeria spp., Salmonella spp., Vibrio cholera*; viral contamination such as presence of Enterovirus spp., hepatitis A, norovirus spp., and rotavirus spp.; parasitic contamination such as presence of *Giardia* spp. and *Trichinella* spp.; and fungal contamination.

More specifically, the Type III CRISPR/Cas detection system according to the invention may further be used for detection of any organism, as all organisms generate RNA during infection. Said organisms include bacteria such as *Bacillus* species, *Clostridium* species, *Enterobacter* species, *Escherichia* species, *Enterococcus* species, *Klebsiella* species, *Listeria* species, *Legionella* species, *Salmonella* species, *Staphylococcus* species, *Streptococcus* species, and combinations thereof; viruses including DNA viruses such as hepatitis B virus, adenovirus, human papilloma virus; RNA viruses such as Influenza virus, Hepatitis A/C/D/E, polio virus, tobacco mosaic virus, Coronavirus, and HIV; viroids; Archaea; fungi such as *Aspergillus* species, *Ascomycetes* species, *Candida* species; protozoa; and parasites such as *Trypanosoma* species

The use of the Type III CRISPR/Cas detection system according to the invention for detection of RNA will find explicit benefit in a diagnostic setting. Two such diagnostic settings will be elaborated herein below. However, a person skilled in the art will without any doubts be able to find additional diagnostic settings that will benefit from the Type III CRISPR/Cas detection system according to the invention for detection of RNA.

A first example is illustrated by recent global developments. Precision point-of-care (PoC) detection would provide many benefits in the COVID-19 pandemic. The recent SARS-CoV-2 outbreak, resulting in the COVID-19 pandemic, has spread all over the world within months after its discovery. To slow down the spread of the disease, many countries have instituted preventative measures restricting the movement and travel of their population. These restrictions are ultimately to "flatten the curve", with the intention of keeping the pressure on national healthcare systems manageable and to save as many lives as possible. However, some countries apparently have been able to manage the rise of infections much more effectively than others. The limited number of infections and subsequent deaths in, for example, Singapore, South-Korea and Taiwan seems to reflect the success of their rapid response. A major factor in this success was their early widespread deployment of mass testing and subsequent self-quarantine, which other countries did not or could not do. This diagnostic screening program has aided in identifying infected people and isolating them, resulting in a considerably slower spread of the virus, leading to fewer infections and lower mortality. In addition to this, the WHO has expressed the need for decentralized, wide-spread testing to enable a rapid response to emerging pandemics. The most important diagnostics tool used in COVID-19 testing is based on (RT)-PCR, which detects the viral genetic material (RNA). While this test yields reliable results in 4-6 hours, only centralized laboratories are qualified to perform the tests, which requires sample shipping and an expected turnaround time per test of at least 24 hours. Even in developed countries, the recently increased testing capacity does not meet the demands. The only available PoC tests are immunoassays, which do not discriminate between past and current infections, and can not be used for acute-phase diagnosis.

CRISPR-Cas nucleic acid detection diagnostics offer a solution for setting up a decentralized screening platform. CRISPR-Cas based diagnostics have been claimed to be faster than PCR, and cheaper to perform on site (Sheridan, 2020. Nat Biotechnol 38: 382-384). We are developing an innovative, proprietary CRISPR-Cas based PoC COVID-19 diagnostic approach, which will contribute to improving the virus monitoring capacity and hence limit the spread of COVID-19 and other viruses.

A second example is provided by detection of urinary tract infection (UTI). Current UTI diagnostics are not yet up to scratch. Especially in first line healthcare, the general practitioner has to rely on unreliable dipstick results with a positive predictive value of only 61%. This leads to high amounts of treatment of disease-negative patients and unnecessary prescription of antibiotics (Eriksen and Bing-Jonsson, 2016. Forskning 1-42; Available at sykepleien.no/forskning/2016/09/k an-ikke-stole-blindt-pa-urinstiks).

Precision point-of-care diagnosis using the Type III CRISPR/Cas detection system will provide major advantages over the present methods. Due to its ability for specific RNA detection, the Type III CRISPR/Cas detection system allows for quick species identification and detection of antibiotic resistance markers.

Most (70-90%) cases of UTIs are caused by uropathogenic *E*. *coli,* but may also be caused by a range of other pathogens (Flores-Mireles et al., 2015. Nat Rev Microbiol 13: 269-284). Identifying which specific pathogen causes infection will provide additional information to increase the chance of correct administration of antibiotics.

For example, identification of specific pathogens may be based on the expression of specific 16S ribosomal RNA species. Van der Zee et al. (van der Zee et al., 2016. PLOS ONE 11: e0150755) showed that differentiation between seven UTI-causing pathogens can be based on 16S based qPCR. Some examples of the primers that were used are provided in Table 3 (Van der Zee et al., 2016. *Ibid*)*.* The targets of these primers can be adapted to Type III CRISPR/Cas guide sequences. Similarly, PCR primer regions for detection of antibiotic resistance markers can also be adapted to fit Type III CRISPR/Cas based detection.

Another example is provided by detection of *Listeria* in raw dairy. *Listeria monocytogenes* is a well-known food-borne pathogen which can cause fatal listeriosis. *Listeria* has been found to contaminate food such as vegetables, dairy and meat products. Presence of *Listeria* in 1 - 4.4% of all raw milk has been reported in Europe, as well as from 1.1 up to 65% in certain unpasteurized cheeses (Lundén et al., 2004. J Dairy Science 87, E6 - E12). Type III CRISPR/Cas based genetic detection of *Listeria* can be combined with detection of other potential pathogens such as *Escherichia coli, Salmonella typhimurium* and *Campylobacter jejuni.*

Rapid detection of food-borne pathogens using rapid diagnostics could potentially prevent use and consumption of contaminated dairy. Enabling this detection at the farm would prevent transport of contaminated milk to the dairy processing plant and prevent potential spreading of the contamination, decreasing the chance of *listeriosis.*

As in the first example, Type III CRISPR/Cas based RNA detection can be designed based on similar PCR target sequences. Complications may arise in potentially required pre-treatment of milk, as well as the likely required low detection limit.

If necessary, for example to increase detection levels, the methods of the invention for detecting specific RNA sequences can be preceded by amplification of target sequences. Amplification may be performed by any suitable amplification system including, for example, ligase chain reaction (LCR), isothermal ribonucleic acid amplification systems such as nucleic acid sequence-based amplification (NASBA) and cleavage-based signal amplification of RNA, transcription mediated amplification, strand displacement amplification and, polymerase chain reaction (PCR). As is known to a person skilled in the art, RNA preferably is reverse transcribed prior to, or during the amplification reaction.

A preferred amplification reaction is a single tube, isothermal reaction such as NASBA, loop-mediated isothermal amplification (LAMP), helicase-dependent amplification (HDA), recombinase polymerase amplification (RPA) reaction, and nicking enzyme amplification reaction (NEAR). A preferred single tube, isothermal reaction is a RPA) reaction (TwistDx Ltd., Cambridge, UK).

Said single tube, isothermal reaction such as RPA preferably is integrated with the CRISPR-based ribonucleic acid detection system of the invention as a "one pot" reaction system. An advantage of such one pot, or single tube, system is a reduced risk for contamination of the samples, or cross-contamination of different samples.

For detection of cOA by employing a direct cOA detection method, samples may comprise endogenous levels of PPi and Pi. Especially biological fluids such as blood plasma, blood serum, urine and synovial fluid may contain amounts of PPi and Pi of 0.16 - 3.42 microM and 0.31 mM, respectively (blood plasma); of 3.5 microM and 1-1.5 mM, respectively (blood serum); of 1.5 mM Pi (urine) and of 0.1 microM and 0.3 mM, respectively (synovial fluid) (Russell et al., 1970. Lancet 296: 899-902; Silcox and McCarty, 1973. J Clin Invest 52: 1863-1870; Bansal, 1990. Serum Inorganic Phosphorus. In: Clinical Methods: The History, Physical, and Laboratory Examinations (Butterworths); Le, 2008. First aid for the USMLE step 1 2018). These levels may vary over time and in the course of a disease (Armstrong et al., 1975. Clin Chem 21: 104-108).

Pre-treatment of a sample with high endogenous PPi or Pi levels may be performed by removal of small molecules such as Pi and PPi prior to detection of specific RNA sequences. Several methods, including but not limited to the methods listed below can be taken to prevent readout interference by endogenous PPi or Pi levels, including chemical precipitation, dialysis and the use of cation exchange columns.

Removal of small molecules such as Pi and PPi may be performed, for example, by filtering of the samples over a filter with a molecular weight cut-off of less than 1,000, preferably less than 500. The molecular weight cut-off is defined as the lowest molecular weight (in Daltons) at which greater than 90% of a solute with a known molecular weight is retained by the membrane.

The influence of endogenous phosphorus species on the test results may also be negated by isolation of genetic material (among which the target sequence) from the patient samples and subsequent application in/on a PPi/Pi free medium. Methods for the isolation of nucleic acid material include, but are not limited to organic extraction, chelex extraction, solid phase extraction, magnetic beads, and/or anion exchange.

In addition to the reduction of endogenous Pi levels, sample preparation steps are also required to solve readout interference by other factors including, but not limited to, proteases in patient samples, salt concentrations, and pH.

**Table 3. Specific qPCR primers as described in van der Zee et al. (van der Zee et al., 2016. PLOS ONE 11: e0150755) for detection of UTI-causing pathogens.**

| Organism | target | | oligonucleotides (5'-3') |
|---|---|---|---|
| *E. coli* | RfaH | F | TACGCCCGCCGTTGAC |
| | RfaH | R | AGCCAGCAGGCGCAAA |
| | RfaH | CY5 | AACAGGACGAATACTGACGCGCCA |
| *Klebsiella spp.* | CopG | F | CGAAGAAGACGGCATGGAAT |
| | CopG | R | CGCAGATCCGGAGGTCATTA |
| | CopG | YY | TCAACGTCAGCTACGCAAGGAGTG |
| | Pgi | F3 | GAAGGTGAAGATGTTCATAATCACG |
| | Pgi | R2 | CGTGAAATCACGCCGTTCAG |
| | Pgi | R3 | GCGTGAAATTAAGCCGTTCAG |
| | Pgi | YY | CATACAGGGCAATCAGCGCGCC |
| *Citrobacter spp.* | MrkC | F1 | ATGTGTATACCAATGGTGAGTGGAA |
| | MrkC | R1 | GCCCATTTTGCCGTCTTTT |
| | MrkC | FAM | CCGCTACGACCTGAATATCACCCGTG |
| | Cfa | F1 | CCTGGTGGCCGGGATT |
| | Cfa | R1 | GTCCAGCGCATTCAGATGAGT |
| | Cfa | R3 | GGCATCAAGCGCATTAAGATGTAT |
| | Cfa | FAM | CCGCAACCCTTTCCGATCTGGAG |
| *Enterobacter spp.* | Xx | F1 | ACAAAGGAGTCGGGATGAGTTC |
| | Xx | R1 | CGACCATTGCTCGTAAGGCT |
| | Xx | FAM | CAATCCCAGGCCAAATCACCGG |
| | Omp | F2 | CCCATGCTTCAGCTTTGTCA |
| | Omp | R2 | CTGCAGGTTACGCTAACTCCAA |
| | Omp | FAM | CGTTGCCGTCACGTTTCTGGTCAA |
| *P. aeruginosa* | OprL | F1 | GCGTCGAGCTGAAGAAGTAAGAAG |
| | OprL | R | GGCTGAGCGCGAGGAA |
| | OprL | CY5 | CCCAAGCACCTGCGTGTCCTGAC |
| *E. faecalis* | RecG | F2 | GCGAAATGGATGTATCAATCATTG |
| | RecG | R | TTTCCATCCATTCTAAAACAGTATCTAACT |
| | RecG | YY | ACACGTTGGATTCGGCCGCC |
| *P. mirabilis* | Hns | F | GCACGTTTAGCACGACCAGTT |
| | Hns | R | TGCCGATGGTATTGATCCAA |
| | Hns | FAM | CGCCAGCAGCTTCAAGCAGGTCA |

| | | | |
|---|---|---|---|
| Abbreviations: F=forward primer. R=reversed primer. CY5, YY and FAM are fluorescent dyes. | | | |

### 4.6 Suitable devices

The CRISPR/Cas-based ribonucleic acid detection system according to the invention preferably is present in a device. Said device preferably comprises one or more detection systems that target one or more specific RNA sequences. Said device may comprise openings such as inlet and outlet ports, for the introduction and extraction of fluids into and from the device. Said openings may be connected to valves, tubes, channels, chambers, syringes and/or pumps The devices may be connected to fluid flow actuators that allow directional movement of fluids within the microfluidic device. Example actuators include, but are not limited to, syringe pumps, mechanically actuated recirculating pumps, electroosmotic pumps, bulbs, bellows, diaphragms, or bubbles intended to force movement of fluids. In certain example embodiments, the devices are connected to controllers with programmable valves that work together to move fluids through the device. Additionally, a heating mechanism may be for incubation of the reaction mixture at a desired temperature.

Said CRISPR/Cas-based ribonucleic acid detection system preferably is present in a biosensor, preferably by using a disposable cartridge. A preferred biosensor provides methods and means for detecting interaction of the CRISPR/Cas-based ribonucleic acid detection system with a target nucleic acid. A preferred biosensor comprises a reusable hand-held reader capable of simple pushbutton operation for automated analysis of samples, and cost-effective disposable cartridges, preferably disposable microfluidic sensor cartridges, that have been functionalized to provide optimal detection and/or quantification of multiple clinically relevant agents such as pathogens. A possible biosensor is a lateral flow test device, for example based on the accumulation of quantifiable substances such as magnetic particles.

In an embodiment, said device or biosensor is a Point of Care (POC) testing device, which is a transportable, portable, and handheld instrument or test kit that allows to collect a sample and obtain the results in a very short period of time at or near the location of the patient so that the treatment plan can be adjusted as necessary. Said device preferably comprises means that allow a rapid, low-cost, and reliable determination of the presence or absence of a target nucleic acid and, preferably also, a quantification of said target nucleic acid.

A POC comprising a CRISPR/Cas-based ribonucleic acid detection system preferably is directed to detecting a limited number of target nucleic acid molecules, including 5 or less target nucleic acid molecules, 4 or less target nucleic acid molecules, 3 or less target nucleic acid molecules, such as 2 target nucleic acid molecules and 1 target nucleic acid molecule. For this, the crRNA ribonucleoprotein complexes are preferably present at discrete positions ribonucleic acid detection system, allowing to determine a level of cOA for each of the individual crRNA ribonucloprotein complexes.

Said array comprising a CRISPR/Cas-based ribonucleic acid detection system preferably targets at least 5 different target nucleic acid molecules, preferably at least different 10 target nucleic acid molecules, preferably at least different 20 target nucleic acid molecules, preferably at least different 50 target nucleic acid molecules, preferably at least different 100 target nucleic acid molecules. Said array comprising a CRISPR/Cas-based ribonucleic acid detection system preferably targets between 2 and 12.000 distinct target nucleic acid molecules.

As will be clear to a person skilled in the art, said different target nucleic acid molecules may all be directed to different organisms such that each of the crRNA molecules is derived from, and is used to detect, a different target organism such as different bacteria, viruses, fungi, protozoa, and/or parasites. Said different crRNA molecules may also be chosen such that a subset of the different crRNA molecules are directed to the same organisms. Said subset may comprise 2 different crRNA molecules, 3 different crRNA molecules, 4 different crRNA molecules, 5 different crRNA molecules. The number of different crRNA molecules that are directed to the same organism preferably is limited to a maximum of 10. It will be clear that detection of a target organism by all different crRNA molecules provides confirmation that the identification of the target organism is correct.

In addition, a specific crRNA molecule may be present in multiple copies in a CRISPR/Cas-based ribonucleic acid detection system according to the invention, for example in multiple wells of a microtiter plate such as a 48 wells plate, a 96 wells plate, a 192 wells plate, a 384 wells plate or a 768 well plate. Detection of a target organism by multiple copies of a crRNA molecule provides confirmation that the identification of the target organism is correct.

### 5. EXAMPLES

### Example 1

### Materials and methods

*Detailed description of the purification of the Cmr complex and expression and purification of recombinant Cmr proteins*
Cas proteins and Cas complexes were purified as described in Staals et al., 2013 (Staals et al., 2013. Mol Cell 52: 135-145).

### In vitro activity assays

RNA substrates (Table 4) were 5' labelled by T4 polynucleotide kinase (NEB) and 5' 32P-y -ATP, after which they were purified from a denaturing PAGE using RNA gel elution buffer (0.5M Sodium acetate, 10mM MgCl₂, 1mM EDTA and 0.1% SDS). *In vitro* activity assays were conducted in TtCmr activity assay buffer (20mM Tris-HCl pH 8.0, 150mM NaCl, 10mM DTT, 1 mM ATP, and 2 mM MgCl₂ ) using the labeled RNA substrate and 400nM TtCmr. Unless stated otherwise, the reaction was incubated at 65°C for 1 hour. RNA loading dye (containing 95% formamide) was added to the samples after incubation boiled at 95° for 5 minutes. The samples were run over a 20% denaturing polyacrylamide gel (containing 7M urea) for about 3-4 hours at 15mA or overnight at a constant of 4mA. The image was visualized using phosphorimaging.

### Complex reconstitution of Cmr46 and Cmr40

First, 3.5 µL crRNA (700 ng) was added to 3.5 µL 1X Cmr buffer (20 mM Tris-HCl pH 8.0, 150 mM NaCl). Subsequently, for Cmr46, the subunits were added to the reaction mixture in a specific order (Cmr3, Cmr2, Cmr4, Cmr5, Cmr6, Cmr1) to a final concentration of 2.5 µM, 2.5 µM, 2.5 µM, 10 µM, 7.5 µM and 2.5 µM respectively, to make up a total reaction volume of 20 µL. The reaction mixture was incubated at 65°C for 30 minutes. Similarily for Cmr40 with the final concentration of the subunits (Cmr3, Cmr2, Cmr4, Cmr5, Cmr6, Cmr1) being 2.5 µM, 2.5 µM, 1.875 µM, 6.66 µM, 7.5 µM and 2.5 µM respectively.

### Direct cOA detection assay

The *in vitro* cOA detection assays were conducted in TtCmr activity assay buffer (20mM Tris-HCl pH 8.0, 150mM NaCl, 10mM DTT, 1 mM ATP, and 0.5 mM MgCl₂) to which Cmr40- or Cmr46-complex (62.5 nM) as well as the RNA substrates (200 nM, listed in Table 4) were added. The reaction was incubated at 65°C for 1 hour after which 0.05 units of pyrophosphatase (ThermoFisher EF0221) were added, followed by an incubation at 25°C for 30 minutes. The signal was visualized using the Malachite Green Phosphate Assay Kit by Sigma-Aldrich (MAK307).

### Electrophoretic mobility shift assay (EMSA)

EMSAs were performed by incubating 400nM endogenous TtCmr complex with labeled target RNAs (Table 4) in Cmr binding buffer (20 mM Tris-HCl pH 8.0, 150 mM NaCl). All reactions were incubated for one hour at 65°C hour before electrophoresis on a native 5% (w/v) polyacrylamide gel (PAGE) for 2.5 hours at 15mA or overnight at a constant of 4mA. The image was visualized via phosphorimaging.

### Results

### RNA cleavage activity of differently sized TtCmr complexes.

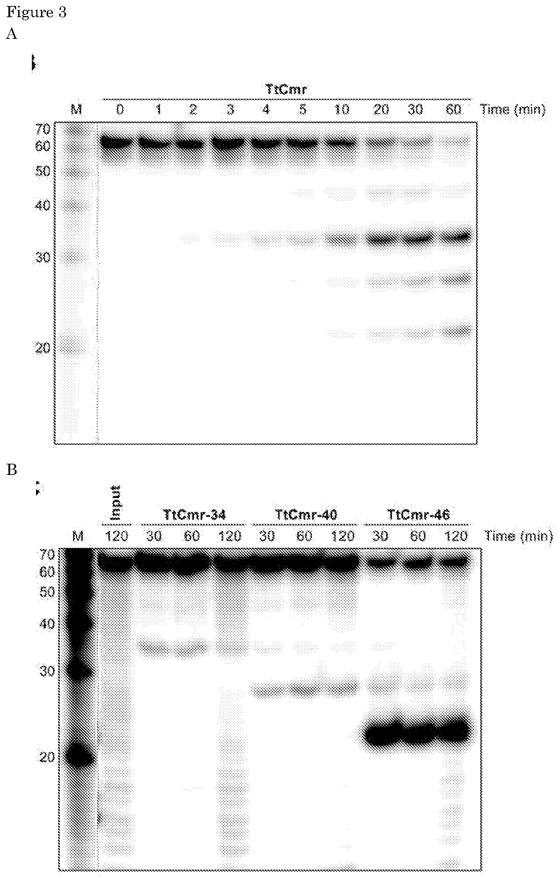

Our previous studies showed that native Cmr complexes purified from *T*. *thermophilus* HB8 were loaded with range of mature crRNA guides of different lengths, and that the crRNA-4.5 (*T. thermophilus* CRISPR array 4, spacer 5) was most abundant (Staals et al., 2013. Mol. Cell 52: 135-145). The endogenous Cmr complex specifically cleaves complementary 5' labeled target RNAs (4.5 target RNA) at 6 nt intervals, resulting in 5' labeled degradation fragments of mostly 21, 27, 33 and 39 nucleotides (Figure 3A). However, the heterogeneous nature of the crRNA-content of the endogenous Cmr complexes (Staals et al., 2013. *Ibid,* Taylor et al., 2015. Science 348: 581-586) complicates the interpretation of these results. Therefore, to further probe the mechanism of target RNA cleavage, we replaced the endogenous Cmr complex with a reconstituted Cmr complex bound to a single crRNA (crRNA-4.5) of different lengths.

**Table 4. Oligonucleotides (target RNAs) used in this study**

| **Oligo name** | **Sequence (5' to 3')** | **Comments** |
|---|---|---|
| P1 | AUUGCGACCCGUAGAUAAGGCGCCCGGGACGACCACG UCAAGGCGCAGUUGC | crRNA 4.5, 46nts (spacer region underlined) |
| P2 | AUUGCGACCCGUAGAUAAGGCGCCCGGGGACGAC | crRNA 4.5, 34nts (spacer region underlined) |
| P3 | AUUGCGACCCGUAGAUAAGGCGCCCGGGACGACCACG UC | crRNA 4.5, 40nts (spacer region underlined) |
| P4 | AUUGCGACCCGUAGAUAAGGCGCCCGGGACGACCACG UCAAGGCG | crRNA 4.5, 46nts (spacer region underlined) |
| P5 | GAACUGCGCCUUGACGUGGUCGUCCCCGGCGCCUUA UCUACGGCCAUCG | Target RNA 1 (underlined part complementary to crRNA 4.5) |
| P6 | GUUGUGCGCCUUGACGUGGUCGUCCCCGGGCGCCUUA UCUACGGGUCCGU | Matched -3/-1 target RNA (underlined part) |
| P7 | GUUGUGCGCCUUGACGUGGUCGUCCCCGGGCGCCUUA UCUACGGCUCGGU | Matched -4/-2 target RNA (underlined part) |
| P8 | GUUGUGCGCCUUGACGUGGUCGUCCCCGGGCGCCUUA UCUACGGCACGCU | Matched -5/-3 target RNA (underlined part) |
| P9 | GAACUGCGCCUUGACGUGGUCGUCCCCGGGCGCCUUA UCUACGCCCAUCG | Mismatched +1 RNA target (underlined part) |
| P10 | GAACUGCGCCUUGACGUGGUCGUCCCCGGGCGCCUUA UCUACCGCCAUCG | Mismatched +2 RNA target (underlined part) |
| P11 | GAACUGCGCCUUGACGUGGUCGUCCCCGGGCGCCUUA UCUAGGGCCAUCG | Mismatched +3 RNA target (underlined part) |
| P12 | GAACUGCGCCUUGACGUGGUCGUCCCCGGGCGCCUUA UCUUCGGCCAUCG | Mismatched +4 RNA target (underlined part) |
| P13 | GAACUGCGCCUUGACGUGGUCGUCCCCGGGCGCCUUA UCAACGGCCAUCG | Mismatched +5 RNA target (underlined part) |
| P14 | GAACUGCGCCUUGACGUGGUCGUCCCCGGCGCCUUA UGUACGGCCAUCG | Mismatched +6 RNA target (underlined part) |
| P15 | GAACUGCGCCUUGACGUGGUCGUCCCCGGGCGCCUUA ACUACGGCCAUCG | Mismatched +7 RNA target (underlined part) |
| P16 | GAACUGCGCCUUGACGUGGUCGUCCCCGGCGCCUUA UCAUGCCCCAUCG | Mismatched +1-5 RNA target (underlined part) |
| P17 | GAACUGCGCCUUGACGUGGUCGUCCCCGGCGCGAAU ACUACGGCCAUCG | Mismatched +7-11 RNA target (underlined part) |
| P18 | GAACUGCGCCUUGACGUGGUCGUCCCCCCGCCCUUA UCUACGGCCAUCG | Mismatched +13-17 RNA target (underlined part) |
| P19 | GAACUGCGCCUUGACGUGGUCCAGGCGGCGCCUUA UCUACGGCCAUCG | Mismatched +19-23 RNA target (underlined part) |
| P20 | GAACUGCGCCUUGACCACCACGUCCCCGGCGCCUUAU CUACGGCCAUCG | Mismatched +25-29 RNA target (underlined part) |
| P21 | GAACUGCGCGAACUCGUGGUCGUCCCCGGCGCCUUA UCUACGGCCAUCG | Mismatched +31-35 RNA target (underlined part) |
| P22 | CUUAUCUACGG | 1-11 base pairing target RNA |
| P23 | GGGCGCCUUAU | 7-17 base pairing target RNA |
| P24 | GUCCCCGGGCG | 13-23 base pairing target RNA |
| P25 | GUGGUCGUCCC | 19-29 base pairing target RNA |
| P26 | CUUGACGUGGU | 25-35 base pairing target RNA |
| P27 | GAACUGCGCCUUGACGUGGUCGUCCCCGGCGCCUUA UCUACGGCCAUCG | Target RNA 2 (underlined part complementary to crRNA 4.5) |
| P28 | GUUGUGCGCCUUGACGUGGUCGUCCCCGGGCGCGUUA UCUACGGCAGCGU | Mismatched +11 RNA target (underlined part) |
| P29 | GUUGUGCGCCUUGACGUGGUCGUCCCCCGGCGCCUUA UCUACGGCAGCGU | Mismatched +17 RNA target (underlined part) |
| P30 | GUUGUGCGCCUUGACGUGGUCCUCCCCGGGCGCCUUA UCUACGGCAGCGU | Mismatched +23 RNA target (underlined part) |
| P31 | GUUGUGCGCCUUGACCUGGUCGUCCCGGGCGCCUUA UCUACGGCAGCGU | Mismatched +29 RNA target (underlined part) |
| P32 | GUUGUGCGCGUUGACGUGGUCGUCCCGGGCGCCUUA UCUACGGCAGCGU | Mismatched +35 RNA target (underlined part) |

We chose to include crRNA lengths of 34 (TtCmr-34), 40 (TtCmr-40) and 46 nt (TtCmr-46) based on their abundance in our previous RNA sequencing data (Staals et al., 2013. *Ibid*). Opposed to the mixture of 5' labeled degradation products observed with the endogenous complex, each of the reconstituted complexes yielded defined degradation products of mostly one size (Figure 3B). This is consistent with the idea that the length of the complex is determined by the length of the crRNA, with bigger complexes (e.g. TtCmr-46) cleaving more closely to the 5' (labeled) end of the target RNA. Smaller complexes (i.e. TtCmr-40 and TtCmr-34) lack one or two Cas7-Cas11 (Cmr4-Cmr5) backbone segments respectively and therefore cleave the target RNA at more distal locations (further away from the 5' label), resulting in larger degradation products. These results show that the population of endogenous TtCmr complexes is a heterogenous mixture of bigger and smaller complexes, cleaving their cognate target RNAs at different positions.

### The seed sequence of TtCmr

To investigate the significance of these type III complexes with different stoichiometries, we set up activity assays to probe for differences in targeting and seed requirements. In the structurally similar type I effector complexes (i.e. the Cascade complex) targeting is governed by two factors: the PAM (protospacer adjacent motif) and the seed (Mojica et al., 2009. Microbiol 155: 733-740; Semenova et al., 2011. Proc Natl Acad Sci U S A 108: 10098-10103; Wiedenheft et al., 2011. Proc Natl Acad Sci U S A 108: 10092-10097). In type III systems however, self-discrimination is conferred by a so-called rPAM, which checks for complementarity between the 8 nt 5' handle of the crRNA (referred to as nucleotides -8 to -1) and the corresponding 3' region flanking the protospacer [Elmore et al., 2016. Genes Dev 2016. 30: 447-459; Kazlauskiene et al., 2016. Mol Cell 62: 295-306.; Marraffini and Sontheimer, 2010. Nature 463: 568-571]. In case of complementarity, the DNase activity of Cas10 is abolished. Since TtCmr is devoid of DNase activity (Staals et al., 2013. *Ibid*), due to a N-terminally truncated Cas10 protein lacking the HD domain (data not shown), we tested whether RNase activity is affected by target RNAs with matches to the 5' handle of the crRNA. However, the activity assays showed that these substrates had no discernable effect on RNA cleavage activity, suggesting that RNA targeting by TtCmr does not check for targeting of self RNAs (e.g. antisense transcripts from the CRISPR array), similar to other type III systems (Tamulaitis et al., 2014. Mol Cell 56: 506-517; Samai et al., 2015. Cell 161: 1164-1174).

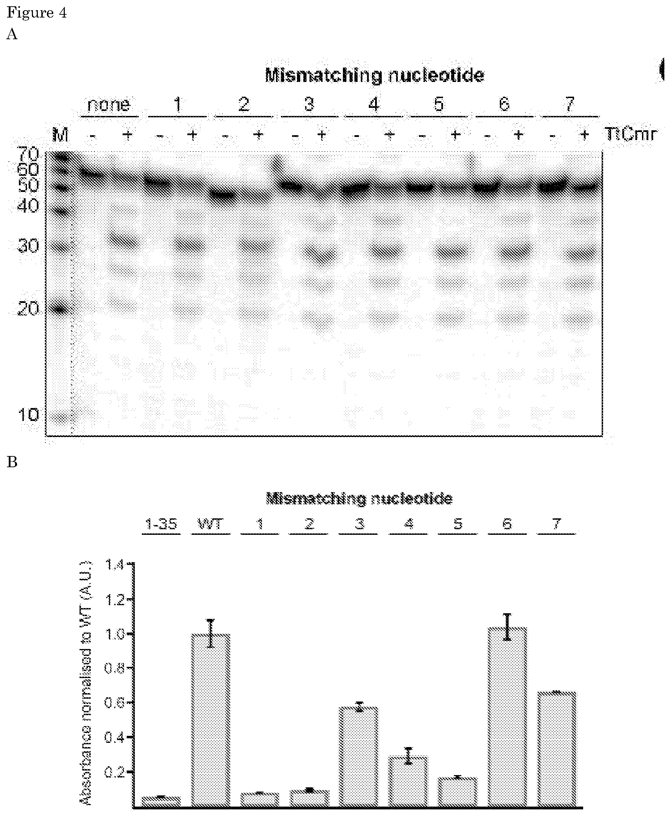

Next, we tested whether TtCmr utilizes a seed similar to type I systems by setting up activity assays with mutations in the first 8 nt of the guide portion of the crRNA (the non-repeat segment of the crRNA base pairing with the protospacer). The results showed that RNA targeting was not affected by these mutations, although a mismatch at position 5 blocked abolished the adjacent cleavage site, as demonstrated by the missing 39 nt degradation product (Figure 4A, B). As demonstrated in Figure 4A, mutations in the first 8 nt of the guide portion of the crRNA do not affect target RNA degradation. However, its effect on cOA production and the subsequent nonspecific RNA degradation was yet to be elucidated. More insight into the effect of single nucleotide mismatches on the production of cOA was achieved by employing a novel assay.

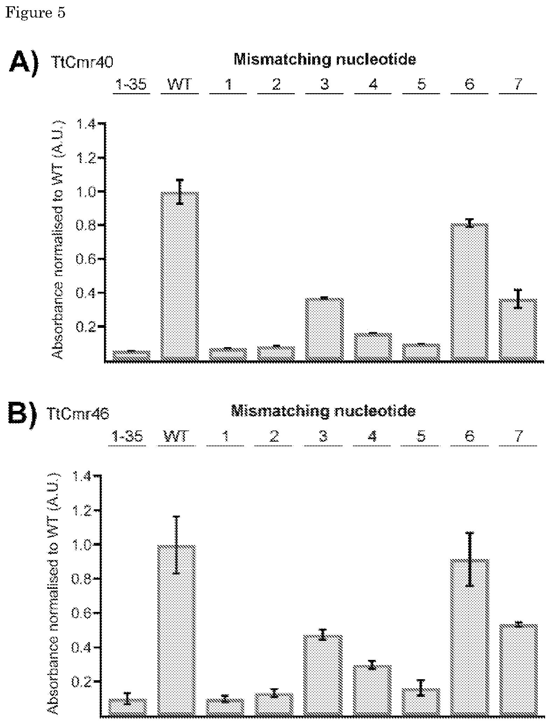

For the endogenous TtCmr complex as well as the two reconstituted Cmr-complexes (46nt & 40 nt, Figure 5) the results clearly demonstrate the importance of having complementarity at nt positions 1, 2 and 5 for the production of cOA (Figure 3). The cOA production is affected to a lesser degree by a mismatch at nt position 4 when compared to 1, 2 and 5. Strikingly, mismatches at nt position 3, 6 and 7 appear to have little impact on the production of the second messenger molecule.

These results suggest that full complementarity is unnecessary in this region and that the seed might be located in a different region instead. Indeed, earlier work on the type IIIB Cmr complex from *Sulfolobus islandicus* (Peng et al., 2015. Nucleic Acids Res 43: 406-417) showed strict base-pairing requirements at the 3' end of the crRNA.

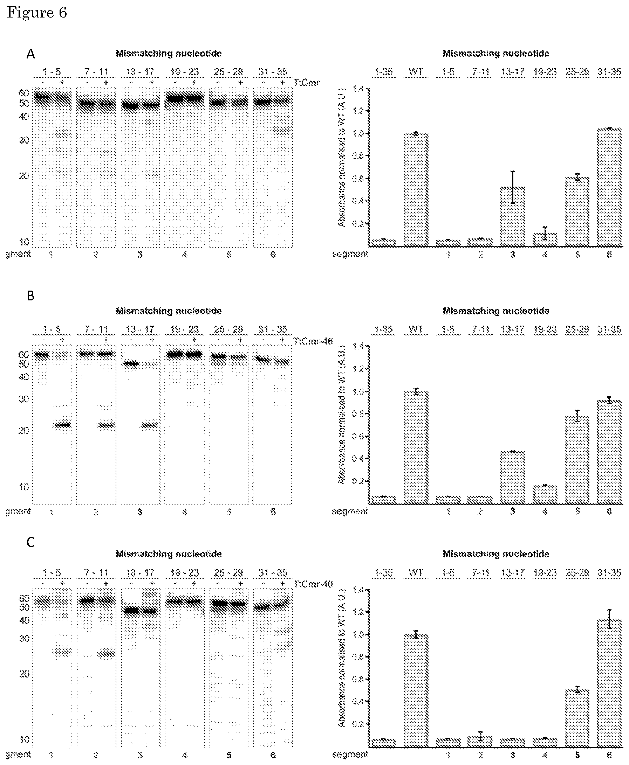

To investigate this possibility, we performed activity assays with the endogenous TtCmr complex and RNA targets with different mismatching segments (Figure 6A). In agreement with the previous findings, RNA targets with mismatches in the first segment (nucleotides 1-5) did not interfere with target degradation, despite skipping one cleavage site downstream of the mismatched segment. However, the effect on cOA production is very significant, abolishing it almost completely (Figure 6). For the second and third mismatched segments (nucleotides 7-12 and 13-17), both the up- and downstream cleavage sites were skipped, whereas the other cleavage sites were unaffected. The second segment mismatch completely abolished the production of cOA while still a significant amount of cOA production was observed in the third segment mismatch. When mismatches were introduced in the fourth and fifth segments (nucleotides 19-23 and 25-29) however, RNA degradation was completely abolished while cOA production was only affected in the fourth segment mismatch. Mismatches in the last segment (nucleotides 31-35) had no effect on RNA degradation or cOA production other than skipping the upstream cleavage site. This indicates that base pairing in the region spanning the fourth and fifth segments are crucial for target recognition and degradation while also playing a role in cOA production initiation.

Since the endogenous complex is a mixture of longer and shorter complexes, we switched to using the reconstituted complexes with 46 (TtCmr-46) or 40 nt crRNA (TtCmr-40) in order to pinpoint this crucial region with more precision. The TtCmr-46 complex almost completely mirrored the results obtained with the endogenous complex, showing the sensitivity of RNA degradation to mismatches within the fourth and fifth segments and significant effects on cOA production in the first, second and fourth segment (Figure 5B). However, this essential region appeared to have shifted 1 segment in the TtCmr-40 complex, with strict base pairing requirements in the third and fourth segments (Figure 5A). Taken together, these results strongly suggest a 3' located seed region within TtCmr that, remarkably, shifts towards that the 5' end of the guide with smaller crRNA (and therefore complex) sizes. We propose that these regions, together or independently, act as a seed sequence in TtCmr.

We hypothesized that this seed sequence is responsible for initiating binding during recognition of a complementary RNA target. For this reason, we tested the binding affinity of the same mismatched RNA targets in an EMSA using the endogenous TtCmr complex (Figure 6A). In accordance with the activity assays, we observed that targets with mismatches in the first three segments (nucleotides 1-5, 7-11, and 13-17) did not interfere with binding to the TtCmr complex and migrated similar to the fully complementary RNA target control. Mismatches in the fourth and fifth segments (nucleotides 19-23 and 25-29) however substantially affected the migration TtCmr-target RNA tertiary complex on the gel. Although the migration was different from the unbound state, we anticipate that this might represent partial binding to the other downstream (complementary) parts of the target RNA.

To further establish that base pairing is initiated at the 3' end of the crRNA, we designed short 11 nt target RNAs in an EMSA. Whereas targets complementary to the 5' parts of the crRNA were unable to base pair with the crRNA, the targets base pairing with the proposed 3' seed region were able to bind (Figure 6B). These results indicated that the 5' end of the guide is somehow shielded from base pairing interactions with its cognate target RNA. Rather, crRNA:target duplex formation appears to be initiated at the 3' end of the guide before propagating towards the 5' end.

We previously solved the cryo-EM structures of differently sized apo- and ssRNA target bound TtCmr complexes (Taylor et al., 2015. Science 348: 581-585). We observed that the complex undergoes a concerted rearrangement of subunits upon target RNA binding. Most notably, the Cas11 (Cmr5) filament rotates away from the center of the complex, thereby exposing the more 5' located parts of the crRNA in a channel that allows further propagation of the crRNA:target RNA duplex. These observations are in good agreement with the identified seed region in this study. For example, the proposed seed resides in a region of higher accessibility and has interactions with Cmr1, Cmr6, and a Cmr4 thumb. It's also the first segment of crRNA that is adjacent to a Cmr5 from the top. This suggests that initial target binding could begin at the most accessible 'open' region of the Cmr1 head; however, the crucial seed sequence residues are required to initiate the conformational changes within the complex by intercalating between the 'closed' major (Cmr4) and minor (Cmr5) backbones. Once the seed region binds, the channel of TtCmr can open, the rest of the substrate can base pair, thereby propagating the concerted conformational changes.

### Example 2

### Materials and methods

### Cmr46-complex reconstitution

First, 3.5 µL crRNA (700 ng) was added to 3.5 µL 1X Cmr buffer (20 mM Tris-HCl pH 8.0, 150 mM NaCl). Subsequently, the subunits were added to the reaction mixture in a specific order (Cmr3, Cmr2, Cmr4, Cmr5, Cmr6, Cmr1) to a final concentration of 2.5 µM, 2.5 µM, 10 µM, 7.5 µM, 2.5 µM and 2.5 µM, respectively, to make up a total reaction volume of 20 µL. The reaction mixture was incubated at 65°C for 30 minutes.

The target RNA sequence used in the assays comprises the sequence: (GAACUGCGCCUUGA)C(GUGGU)C(GUCCC)C(GGGCG)C(CUUAU)C(UACGGCC AUCG), in which the underlined nucleotides were desoxy-based nucleotides. This target RNA cannot be cleaved by the Cmr-complex.

### Pi detection assay

*The in vitro cOA detection assays were conducted in TtCmr activity assay buffer (20mM Tris-HCl pH 8.0, 150mM NaCl, 10mM DTT, 1 mM ATP, and 0.5 mM MgCl2*, *2 units thermostable inorganic pyrophosphatase (NEB#M0296S)) to which Cmr-complex (62.5 nM) as well as the RNA substrates (200 nM unless stated otherwise) were added. The reaction was incubated at 65°C for 1 hour unless stated otherwise. The signal was visualized using the Malachite Green Phosphate Assay Kit by Sigma-Aldrich (MAK307).*

### Csx1 Proof of principle experiment

For the Csx1 proof of principle experiment, cOA detection assay was performed as described under"Pi detection assay", except after incubation for 1 h at 65 °C the sample was heat-inactivated at 95 °C for 10 min. After heat inactivation the sample is spun down at 13.000g for 10 min, after which the supernatant was stored at -20 °C. To the reaction mixture with TtCmr activity buffer (20mM Tris-HCl pH 8.0, 150mM NaCl, 10mM DTT, 1 mM ATP, and 0.5 mM MgCl₂, 2 units thermostable inorganic pyrophosphatase (NEB#M0296S) and ~1.5 ug Csx1 (TTHB144), 2 uL of the previously acquired supernatant was added and incubated at 65 °C for 10 min. After incubation either RNase alert (IDT#11-04-03-03) or DNase alert (IDT#11-04-03-04) reagent was added according to the manual provided. This reaction was incubated at 37 °C for 30 min after fluorescence was measured.

### Results

### Sensitivity experiment

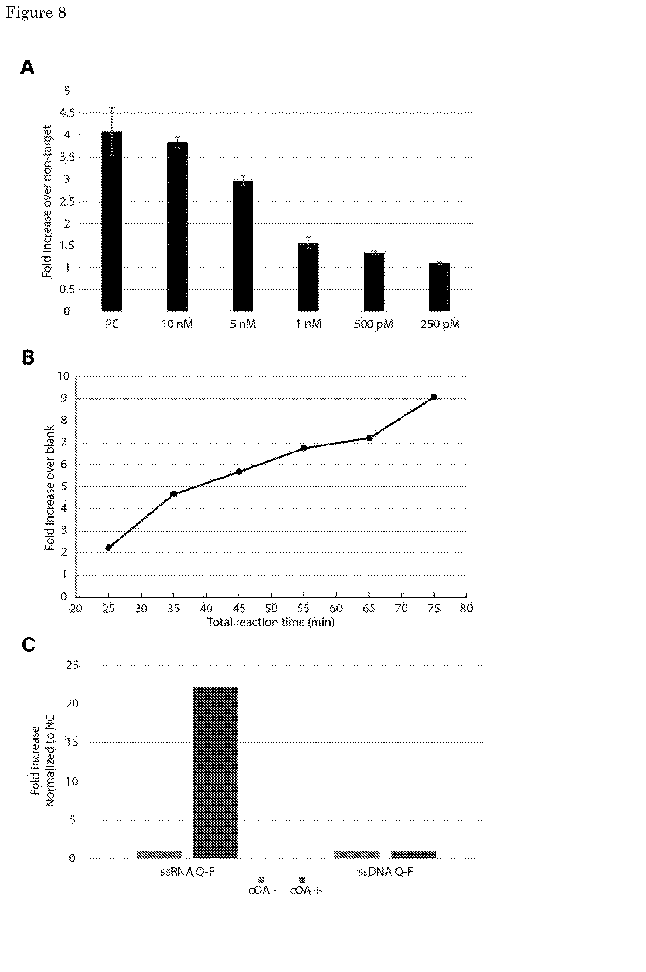

The reaction time (A) was set at 1h and colour development time at 30 min to explore the sensitivity of the system. As shown in Figure 8A, measurable readout (~2 fold increase over non-target) was obtained in the 500 pM-1 nM target RNA range. The strategy of making a mutant subunit lacking the ability to cleave target RNA, to increase effective sensitivity, was emulated by using a target RNA that the complex is unable to cleave.

### Time series experiment

The experiment was set up to determine measurable signal (indicated in fold increase over blank sample) at specified time points. The total reaction time consisted of 2 parts: A) The Pi production reaction upon target RNA addition and B) The colorimetric reaction after addition of the colour reagent. The final output was the fold increase which was measured in absorbance at the end of both reaction times combined. The duration of the total reaction time in the final application was set by the desired measurable threshold (fold increase over blank sample, negative control). In Figure 8B, the dataset was chosen with a set colour reaction time (B) of 15 mins, and a variable Pi production reaction time (A). This was arbitrarily chosen to give an idea of the system.

### Csx1 mediated Proof of principle experiment

This proof of principle experiment indicated in Figure 8C was carried out to proof target RNA induced activation of Csx1. Part of the reaction mixture that was incubated in the presence of target RNA, so that cOA had been produced, was added to a new reaction containing Csx1 and either ssRNA or ssDNA quencher-fluorophore sequences. The results depicted in the bar-graph clearly show ssRNA degradation upon addition of the cOA containing mixture. This indicated that Csx1 can be activated indirectly by the type III CRISPR Cas system upon recognition of target RNA.

### Example 3

### Materials and methods

### Cas proteins and Cas complexes

A Cmr4 D26N cleavage-dead mutant was generated by targeted mutagenesis. All Cas protein encoding sequences were generated as synthetic gBlocks (Integrated DNA Technologies (IDT)) after codon optimization for compatibility with the heterologous expression host E. *coli.* All coding sequences were cloned into expression vectors using standard cloning methods known to a person skilled in the art. Furthermore, a Cmr4 D26N cleavage-dead mutant was created by targeted mutagenesis of the Cmr4 gBlock. Expression vectors contained a N- or C-terminal Strep-tag, optionally a TEV cleavage site, a bicistronic design element, a T7 promoter sequence, a terminator sequence, an p15A low copy origin of replication, and a kanamycin resistance marker. The expression vector itself was derived from the p15A cloning vector (Addgene Plasmid #41187). All expression vectors are transformed to *E*. *coli* BL21(DE3) for protein expression.

Recombinant strains were cultured in 2 L Lysogeny Broth (LB) medium, grown until OD600 of ~0.6 was reached. The culture was placed on ice for 1 hour after which isopropyl β-D-1-thiogalactopyranoside was added to a final concentration of 0.2 mM. The culture was subsequently incubated at 18 °C for ~16 hours (overnight). The cells were harvested (5.000g for 10 min.) and lysed in Buffer A (100 mM Tris-HCl, 150 mM NaCl, pH8.0) by sonication and subsequently spun down at 30.000g for 45 min. The clarified lysate was filtered and run over a Buffer A pre-equilibrated StrepTrap FPLC column (GE Healthcare, Chicago, IL). After washing the column with Buffer A until no more protein was present in the flow through, the protein of interest was eluted using Buffer B (100 mM Tris-HCl, 150 mM NaCl & 2.5 mM D-desthiobiotin). If required, the protein was cleaved from the affinity tag by addition of TEV protease (Genscript Cat. No. Z03030) and left to incubate overnight at 4 °C. The protein of interest was separated from the mixture by a HisTrap affinity chromatography step, from which the flow through was collected. If required, an additional size exclusion chromatography was added to achieve higher purity.

### Cmr46-complex and dCmr46-complex reconstitution

First, 3.5 µL crRNA (700 ng) was added to 3.5 µL 1X Cmr buffer (20 mM Tris-HCl, pH 8.0, 150 mM NaCl). Subsequently, the subunits were added to the reaction mixture in a specific order (Cmr3, Cmr2, Cmr4 or Cmr4 D26N, Cmr5, Cmr6, Cmr1) to a final concentration of 2.5 µM, 2.5 µM, 10 µM, 7.5 µM, 2.5 µM and 2.5 µM, respectively, to make up a total reaction volume of 20 µL. The reaction mixture was incubated at 65°C for 30 minutes.

### Indirect cOA detection assay

The *in vitro* cOA detection assays were conducted in TtCmr activity assay buffer (20 mM Tris-HCl pH 8.0, 150 mM NaCl, 10 mM DTT, 1 mM ATP, and 0.5 mM MgCl2) to which reconstituted Cmr46-complex (62.5 nM), RNA substrates at variable concentrations (listed in Table 4 or Table 5), Csx1 (1 uM), and 0.5 uL RNaseAlert QC system (Thermo Scientific, prepared according to manual) were added. The reaction was incubated at 65°C in a qPCR thermocycler, measuring fluorescence output at 2 min intervals (495/520nm excitation/extinction), or by incubation at 65 °C in a Genie III (Optigene), measuring fluorescence output at 15-second intervals (495/520nm excitation/extinction). Measurements at varying concentrations of target RNA was used to determine the sensitivity range of the system.

### Results

### Confirming ability of target RNA binding for dCmr46 and subsequent Cmr2/Cas10 activation

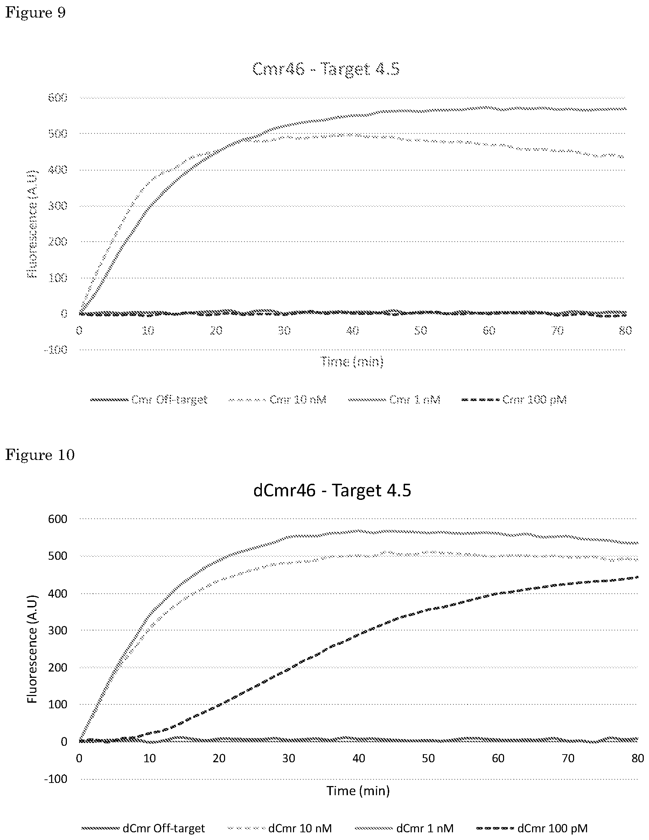

A Cmr4 D26N mutant was generated as it was hypothesized to only affect the cleavage capacity of the complex, but not it's binding capacity or the subsequent activation of the Cmr2/Cas10 subunit. To prove this, the target binding capacity was measured using the previously described indirect cOA visualisation method. Indeed, the mutated protein did not abolish target binding of the full-length Type IIIB complex (Cmr46) (Figures 9, 10).

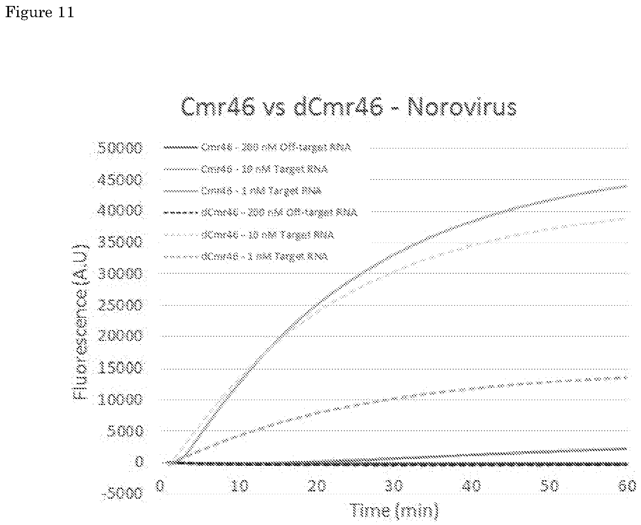

Moreover, the full length catalytically dead Type IIIB complex (dCmr46) was found to increase target RNA sensitivity, when compared to wild-type Type IIIB complex (Figures 9, 10). Additionally, the signal output of the assay indicates that dCmr46 produced more cOA in the same timeframe, when compared to wild-type Cmr46 (Figure 11). We aim to exploit these characteristics of Cmr4 D26N for diagnostic purposes, in which we apply this mutation in *in vitro* assays for higher target sensitivity. This gives the benefit of detecting lower concentrations of target material in assay samples, or lowering the required level of pre-amplification to reach the limit of detection.

**Table 5. RNA oligos used during indirect cOA detection assays**

| Oligo name | Sequence (5' to 3') | Comments |
|---|---|---|
| P33 | AUUGCGACCCGUAGAUAAGGC GCCCGGGGACGACCACGUCAA GGCG | crRNA 4.5, 46nts |
| P34 | GAACUGCGCCUUGACGUGGU CGUCCCCGGCGCCUUAUCUA CGGCC AUCG | Target RNA 1 (underlined 38nts complementary to crRNA 4.5). This RNA was also used as off-target in the norovirus assays. |
| P35 | AUUGCGACACUUAAGCCUUCA GUUGGAAAAUUUGGUGGGAC UGCUG | crRNA norovirus, 46nts |
| P36 | GCAAGUAAUUCUCGCGGGAA CGCGUUCACCGCCGGGAAGAU CAUAU | Target RNA norovirus (underlined 38nts complementary to crRNA norovirus) |
| | UUGCAGCAGUCCCACCAAAUU UUCCAACUGAAGGCUUAAGUC CUAGCC | |
| | AGGUCACUAUGUUCCCCCAUA | |
| P37 | CGUAAACGACGGCCAGUGCCA AGCUUACUAUACAACCUACUA CCUCAUCA | Off-target RNA |

### Example 4. Type-IIIA system (Csm) from Thermus thermophilus

### Materials and methods

### cOA detection assay

The *in vitro* cOA detection assays were conducted in TtCmr activity assay buffer (20mM Tris-HCl pH 8.0, 150mM NaCl, 10mM DTT, 1 mM ATP, and 0.5 mM MgCl₂) to which endogenous Csm-complex (~2 µg; Staals et al., 2014. Mol Cell 56: 518-530) as well as RNA substrates (200 nM, listed in Table 4) were added. The reaction was incubated at 65°C for 1 hour after which 0.05 units of pyrophosphatase (ThermoFisher EF0221) was added, followed by an incubation at 25°C for 30 minutes. The signal was visualized using the Malachite Green Phosphate Assay Kit by Sigma-Aldrich (MAK307). The experiment was carried out according to the layout indicated in Table 6.

**Table 6. Reaction schematic for direct cOA detection assay using endogenous Type IIIA Csm RNP complex. Reactions A to D are set up according to the presented composition.**

| | **A** | **B** | **C** | **D** |
|---|---|---|---|---|
| Csm complex | + | + | + | + |
| Target RNA | - | - | + | + |
| Pyrophosphatase | - | + | - | + |

### Results

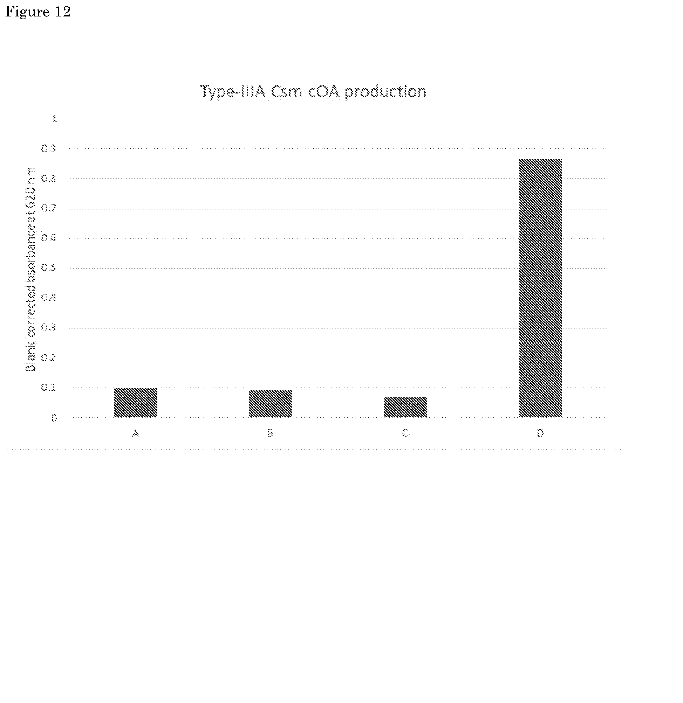

Figure 12 shows the results of the Type IIIa Csm cOA production assay. This Type-III CRISPR/Cas complex also shows cOA production after addition of a complimentary target RNA.

### Example 5. One-pot SARS-CoV-2 detection

### Materials & Methods

### Design of Type IIIB Cmr crRNA and RPA primers

Based on reports by the World Health Organization (WHO) and Centers for Disease Control and Prevention (CDC), the N gene was identified as a suitable target for SARS-CoV-2 detection. A crRNA was designed for a region in the N gene, referred to as N3 by the CDC, which is conserved in multiple SARS-CoV-2 - like coronaviruses:
crRNA_N3:
   5'-AUUGCGACGCAGCATTGTTAGCAGGATTGCGGGTGCCAATGTGATC 3'

To amplify the N3 region, DNA primers for RPA were designed according to parameters proposed by TwistAmp Liquid Basic kit (TwistDx Ltd, Maidenhead, UK). Additionally, a T7 promoter sequence was added to the 5' of the forward primer for *in vitro* transcription. By screening multiple candidates for successful amplification, we identified the best performing primer pair:
nCOV_N3_RPA_F1: 5' 
GGCATAATACGACTCACTATAGGGTCTGATAATGGACCCCAAAATCAGCGAAA
T 3'
nCOV_N3_RPA_R1: 5' CTCCATTCTGGTTACTGCCAGTTGAATCTG 3'

The Cmr46 complex was reconstituted as described previously using crRNA_N3.

The SARS-CoV-2 detection assay combines RPA and Type IIIB Cmr detection in a single reaction. Synthetic SARS-CoV-2 genome was used as target RNA. This genome was provided by Twist Biosciences (South San Francisco, CA) as six nonoverlapping 5 kb RNA fragments at approximately 10⁶ copies per microliter. The reference genome was created based on the isolate Wuhan-Hu-1 (GenBank ID: MN908947.3).

TwistAmp Liquid Basic kit (TwistDx) provided reagents for amplification by RPA. To allow working with small sample volumes, every RPA reaction mixture as provided by the supplier was divided in four equal parts. Additionally, oligonucleotide primers, components for reverse transcriptase, and T7 *in vitro* transcription were incorporated into these mixtures: 1.5 µL primer mixture (10 mM; Integrated DNA Technologies), 0.5 µL M-MLV RT (100 U/µL ; Invitrogen), 0.5 µL DTT (50 mM; Invitrogen), 0.25 µL Murine RNase inhibitor (40 U/µL; New England Biolabs (NEB)), 0.5 µL T7 polymerase (50 U/µL; NEB), 2.25 µL dNTP solution mix (10 mM; NEB), and 2.25 µL NTP solution mix (20 mM; NEB). Next to the RPA mixture, a SARS-CoV-2 Cmr mixture was created by combining 0.125 µL RNaseAlert QC system (Thermo Scientific, dried pellet as provided by manufacturer resuspended in 100 µL instead of 1 mL), 1 µL Csx1 (10 µM), Cmr46 complex to a final reaction mixture concentration of ~60 nM, and ATP to final reaction mixture concentration of 1 mM. After mixing both reaction solutions, 16.5 µL RPA mixture was combined with 4.25 µL SARS-CoV-2 Cmr mixture, and 1 µL of SARS-CoV-2 synthetic genome template. Multiple mixtures were created containing genome template in a range from 100,000 copies/µL to 10 copies/µL. Reactions were incubated at 37 °C for 30 min and 65 °C for 45 min in a Genie III (OptiGene). The instrument performed fluorescent readout starting from 30 min onwards at 495/520 nm (excitation/emission).

### Results

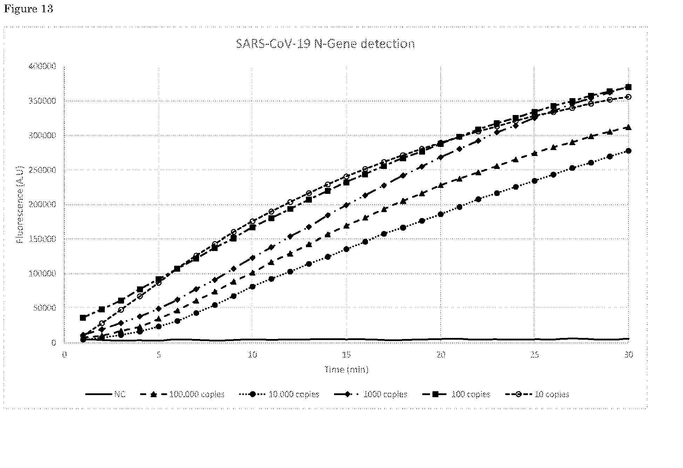

SARS-CoV-2 detection assay combines amplification of target genetic material with detection in a single reaction. This one-pot reaction mixture was incubated at 37 °C for 45 minutes (pre-amplification), after which the temperature was increased to 65 °C (signal production). In Figure 13, the data from the incubation period of the SARS-CoV-2 synthetic genome is depicted. The results show that within minutes of incubation at 65 °C the signal increases significantly compared to the negative control (NC). It is estimated that the pre-amplification time can be reduced further, since signal production can generally already be observed after 5 minutes of incubation at 65 °C.

### Example 6. Validation of one pot SARS-CoV-2 detection

### Materials and methods

In collaboration with Wageningen Bioveterinary Research (WBVR) a small-scale trial was performed to validate the one-pot Type III detection assay for SARS-CoV-2 in mink samples. RNA extraction was performed on rectal swab samples of 4 COVID-positive minks (GenBank IDs MT396266, MT457390 and MT457399) using a Direct Zol RNA miniprep kit (Zymo Research). Sample was eluted in 100 µL RNase free water, of which 5 µL was used as template for the detection assay. TwistAmp^{™} nfo Kit (TwistDx) provided reagents for amplification by RPA. To allow working with small sample volumes, every RPA reaction mixture as provided by the supplier, was divided in four equal parts. Provided Primer Free Rehydration buffer was combined with 1.05 µL primer mixture per reaction (5 mM Forward and Reverse; Integrated DNA Technologies), and subsequently used to rehydrate the provided RPA pellets. Additionally, a Type IIIB Cmr mixture was created by combining 4.5 µL reconstituted Cmr46 complex (to a final reaction mixture concentration of ~60 nM, loaded with N3 gene crRNA), 0.125 µL RNaseAlert QC system (Thermo Scientific, dried pellet as provided by manufacturer resuspended in 100 µL instead of 1mL), 0.25 µL ATP (80 mM; Sigma Aldrich), 1 µL Csx1 (10 µM), 0.5 µL M-MLV RT (100 U/µL ; Invitrogen), 0.5 µL DTT (50 mM; Invitrogen), 0.25 µL Murine RNase inhibitor (40 U/µL; New England Biolabs (NEB)), 0.5 µL T7 polymerase (50 U/µL; NEB), and 2 µL NTP solution mix (20 mM; NEB). For each assay reaction, 8.425 µL RPA mixture was combined with 5.625 µL Cmr mixture, 0.625 µL MgOAc (280 mM, TwistDx), and 5 µL template or RNase free MQ, to a total of 19.675 uL. As a positive control, 5 µL of 100 copies/µl. of SARS-CoV-2 synthetic genome (see Example 5) was used. Reaction mixtures were incubated in a Genie III (OptiGene) spectrophotometer at 37 °C for 45 minutes and subsequently at 65 °C for 30 minutes. Data collection is done during the 65 °C incubation period, measured for fluorescence at 495 nm / 520 nm (excitation / emission) with 15 second intervals.

### Results

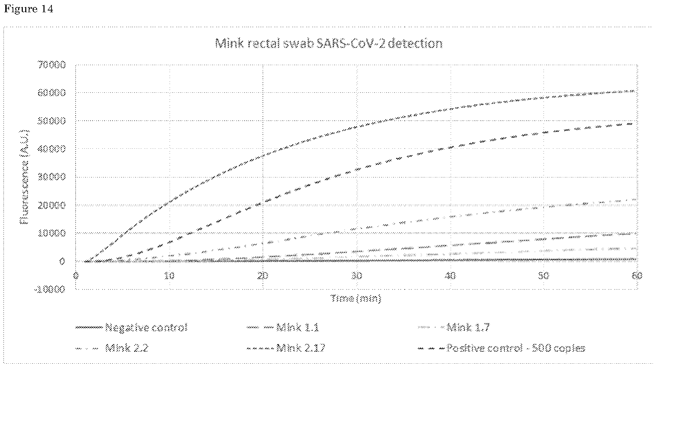

Since the first outbreak of SARS-CoV-2 in mink farms in the Netherlands at the end of April 2020, WBVR has been responsible for all diagnostic testing of minks in The Netherlands. Extracted RNA samples were provided and subsequently used in diagnostic assays which combined amplification of target genetic material with detection in a single reaction. This one-pot reaction mixture was incubated at 37 °C for 45 minutes (pre-amplification), after which the temperature was increased to 65 °C (signal production). Data from the incubation period of the mink SARS-CoV-2 samples are depicted in Figure 14. These results clearly show that detection of SARS-CoV-2 is possible within 30 minutes of incubation at 65 °C, showing the applicability of this technology on real-world complicated sample matrices.

## Claims

1. A method for determining presence or absence of a target nucleic acid molecule in a sample, wherein the method is for detection of bacterial, fungal, archaeal, protest, protozoal, eukaryotic, viral and viroidal pathogens, for detection of beneficial organisms, or for detection and diagnosis of genetic alterations or traits that are expressed as RNA molecules, in human, animals and plants, comprising:
amplification of the target nucleic acid molecule present in the sample, and
providing the sample with a clustered regularly interspaced short palindromic repeats (CRISPR) based ribonucleic acid detection system derived comprising:
a) an effector complex comprising a Type III CRISPR-associated effector protein (Cas) and at least one CRISPR RNA (crRNA) that binds to a target nucleic acid molecule;
b) means for directly or indirectly determining a level of cyclic oligoadenylate (cOA);
incubating the sample under conditions that allow binding of the crRNA to its target nucleic acid molecule; and
directly or indirectly determining a level of cyclic oligoadenylate (cOA), whereby an increase in the determined cOA level, as compared to a control, is indicative of the presence of said target molecule in said sample.

2. The method of claim 1 wherein the Type III Cas is from a thermophilic organism.

3. The method according to claim 2, wherein the sample is incubated at a temperature of between 40 °C and 80 °C, between 50 °C and 70 °C, such as between 55 °C and 65 °C, preferably at about 65 °C.

4. The method according to any preceding claim, wherein the amplification is performed by ligase chain reaction, isothermal ribonucleic acid amplification system, strand displacement amplification, polymerase chain reaction, loop-mediated isothermal amplification, helicase-dependent amplification, recombinase polymerase amplification reaction, or nicking enzyme amplification reaction.

5. The method according to claim 4, wherein the target nucleic acid molecule is an RNA sequence.

6. The method according to any one of claims 1 to 5, wherein the RNA is reverse transcribed prior to or during the amplification reaction.

7. The method according to any preceding claim, wherein the Type III Cas is a Type IIIB Cas, preferably a Type IIIB Cmr.

8. The method according to claim 7, wherein the thermophilic organism is *Thermus thermophilus.*

9. The method according to any preceding claim, wherein the detection system further comprises a cOA-dependent, non-specific effector endoribonuclease such as Csx1.

10. The method according to claim 9, wherein the detection system further comprises a detectable substrate for said endoribonuclease.

11. The method according to claim 10, wherein the detectable substrate is a RNA molecule that is tagged with a fluorescent reporter molecule on one end and a quencher on the other end.

12. The method according to any one of claims 9 to 11, wherein the level of activity of cOA is indirectly determined by determining the level of the cOA-dependent, non-specific endoribonuclease by detecting the detectable substrate for said effector endoribonuclease.

13. The method of any one of claims 1 to 12 wherein the amplification and detection is performed in a single one-pot reaction method.

14. The method of claim 13 wherein the amplification is a single tube, isothermal reaction.

## Patentansprüche

1. Verfahren zum Bestimmen eines Vorhandenseins oder Nichtvorhandenseins eines Zielnukleinsäuremoleküls in einer Probe, wobei das Verfahren zum Nachweis von Bakterien-, Pilz-, Archaea-, Protest-, Protozoen-, eukaryotischen, viralen und viroidalen Pathogenen, zum Nachweis von nützlichen Organismen oder zum Nachweis und zur Diagnose von genetischen Veränderungen oder Merkmalen, die als RNA-Moleküle ausgedrückt sind, in Menschen, Tieren und Pflanzen dient, umfassend:
Amplifikation des in der Probe vorhandenen Zielnukleinsäuremoleküls und
Versehen der Probe mit einem auf Clustered Regularly Interspaced Short Palindromic Repeats (CRISPR) basierten Ribonukleinsäure-Nachweissystem, abgeleitet umfassend:
a) einen Effektorkomplex, der ein Typ-III-CRISPR-assoziiertes Effektorprotein (Cas) und zumindest eine CRISPR-RNA (crRNA) umfasst, die an ein Zielnukleinsäuremolekül bindet;
b) Mittel zum direkten oder indirekten Bestimmen eines Niveaus an zyklischem Oligoadenylat (cOA);
Inkubieren der Probe unter Bedingungen, die Bindung der crRNA an ihr Zielnukleinsäuremolekül ermöglichen; und
direktes oder indirektes Bestimmen eines Niveaus an zyklischem Oligoadenylat (cOA), wobei eine Erhöhung des bestimmten cOA-Niveaus verglichen mit einer Kontrolle auf das Vorhandensein des Zielmoleküls in der Probe hinweist.

2. Verfahren nach Anspruch 1, wobei das Typ-III-Cas von einem thermophilen Organismus stammt.

3. Verfahren gemäß Anspruch 2, wobei die Probe bei einer Temperatur zwischen 40 °C und 80 °C, zwischen 50 °C und 70 °C, wie etwa zwischen 55 °C und 65 °C, bevorzugt bei etwa 65 °C, inkubiert wird.

4. Verfahren gemäß einem vorhergehenden Anspruch, wobei die Amplifikation durch Ligase-Kettenreaktion, isothermes Ribonukleinsäure-Amplifikationssystem, Strand-Displacement-Amplifikation, Polymerase-Kettenreaktion, Loop-vermittelte isotherme Amplifikation, Helikase-abhängige Amplifikation, Rekombinase-Polymerase-Amplifikationsreaktion oder Nicking-Enzyme-Amplifikationsreaktion durchgeführt wird.

5. Verfahren gemäß Anspruch 4, wobei das Zielnukleinsäuremolekül eine RNA-Sequenz ist.

6. Verfahren gemäß einem der Ansprüche 1 bis 5, wobei die RNA vor oder während der Amplifikationsreaktion revers transkribiert wird.

7. Verfahren gemäß einem vorhergehenden Anspruch, wobei das Typ-III-Cas ein Typ-IIIB-Cas ist, bevorzugt ein Typ-IIIB-Cmr.

8. Verfahren gemäß Anspruch 7, wobei der thermophile Organismus *Thermus thermophilus* ist.

9. Verfahren gemäß einem vorhergehenden Anspruch, wobei das Nachweissystem ferner eine cOA-abhängige, nichtspezifische Effektor-Endoribonuklease wie etwa Csxl umfasst.

10. Verfahren gemäß Anspruch 9, wobei das Nachweissystem ferner ein nachweisbares Substrat für die Endoribonuklease umfasst.

11. Verfahren gemäß Anspruch 10, wobei das nachweisbare Substrat ein RNA-Molekül ist, das an einem Ende mit einem fluoreszierenden Reportermolekül und an dem anderen Ende mit einem Quencher markiert ist.

12. Verfahren gemäß einem der Ansprüche 9 bis 11, wobei das Niveau an Aktivität von cOA indirekt bestimmt wird, indem das Niveau an cOA-abhängiger, nichtspezifischer Endoribonuklease bestimmt wird, indem das nachweisbare Substrat für die Effektor-Endoribonuklease nachgewiesen wird.

13. Verfahren nach einem der Ansprüche 1 bis 12, wobei die Amplifikation und der Nachweis in einem einzelnen Eintopfreaktionsverfahren durchgeführt wird.

14. Verfahren nach Anspruch 13, wobei die Amplifikation eine isotherme Einrohrreaktion ist.

## Revendications

1. Procédé permettant de déterminer la présence ou l'absence d'une molécule d'acide nucléique cible dans un échantillon, dans lequel le procédé est destiné à la détection d'agents pathogènes bactériens, fongiques, archéens, protozoaires, eucaryotes, viraux et viroïdiens, à la détection d'organismes bénéfiques, ou à la détection et au diagnostic d'altérations ou de traits génétiques qui sont exprimés sous forme de molécules d'ARN, chez l'homme, les animaux et les plantes, comprenant :
l'amplification de la molécule d'acide nucléique cible présente dans l'échantillon, et
la fourniture de l'échantillon avec un système de détection d'acide ribonucléique basé sur des répétitions palindromiques courtes groupées et régulièrement espacées (CRISPR) comprenant :
a) un complexe effecteur comprenant une protéine effectrice associée à CRISPR (Cas) de type III et au moins un ARN CRISPR (ARNcr) qui se lie à une molécule d'acide nucléique cible ;
b) un moyen permettant de déterminer directement ou indirectement un niveau d'oligoadénylate cyclique (cOA) ;
l'incubation de l'échantillon dans des conditions permettant la liaison de l'ARNcr à sa molécule d'acide nucléique cible ; et
la détermination directe ou indirecte d'un niveau d'oligoadénylate cyclique (cOA), moyennant quoi une augmentation du niveau de cOA déterminé, par rapport à un témoin, indique la présence de ladite molécule cible dans ledit échantillon.

2. Procédé de la revendication 1, dans lequel le Cas de type III provient d'un organisme thermophile.

3. Procédé selon la revendication 2, dans lequel l'échantillon est incubé à une température comprise entre 40 °C et 80 °C, entre 50 °C et 70 °C, telle qu'entre 55 °C et 65 °C, de préférence à environ 65 °C.

4. Procédé selon une quelconque revendication précédente, dans lequel l'amplification est réalisée par une réaction en chaîne par ligase, un système d'amplification isotherme d'acide ribonucléique, une amplification par déplacement de brin, une réaction en chaîne par polymérase, une amplification isotherme médiée par boucle, une amplification dépendante d'une hélicase, une réaction d'amplification par polymérase recombinase ou une réaction d'amplification par enzyme de coupure.

5. Procédé selon la revendication 4, dans lequel la molécule d'acide nucléique cible est une séquence d'ARN.

6. Procédé selon l'une quelconque des revendications 1 à 5, dans lequel l'ARN est soumis à une transcription inverse avant ou pendant la réaction d'amplification.

7. Procédé selon une quelconque revendication précédente, dans lequel le Cas de type III est un Cas de type IIIB, de préférence un Cmr de type IIIB.

8. Procédé selon la revendication 7, dans lequel l'organisme thermophile est *Thermus thermophilus.*

9. Procédé selon une quelconque revendication précédente, dans lequel le système de détection comprend en outre une endoribonucléase effectrice non spécifique dépendante de cOA telle que Csxl.

10. Procédé selon la revendication 9, dans lequel le système de détection comprend en outre un substrat détectable pour ladite endoribonucléase.

11. Procédé selon la revendication 10, dans lequel le substrat détectable est une molécule d'ARN marquée à une extrémité par une molécule rapporteuse fluorescente et à l'autre extrémité par un désactivateur.

12. Procédé selon l'une quelconque des revendications 9 à 11, dans lequel le niveau d'activité de cOA est déterminé indirectement en déterminant le niveau de l'endoribonucléase non spécifique dépendante de cOA en détectant le substrat détectable pour ladite endoribonucléase effectrice.

13. Procédé de l'une quelconque des revendications 1 à 12, dans lequel l'amplification et la détection sont réalisées dans un seul procédé de réaction monotope.

14. Procédé de la revendication 13, dans lequel l'amplification est une réaction isotherme en tube unique.
